(19) Europäisches Patentamt / European Patent Office / Office européen des brevets

(11) **EP 4 466 381 B1**

(12) **EUROPEAN PATENT SPECIFICATION**

(45) Date of publication and mention of the grant of the patent:
**05.08.2026 Bulletin 2026/32**

(21) Application number: **23715681.5**

(22) Date of filing: **24.02.2023**

(51) International Patent Classification (IPC):
**C12Q 1/6886** *(2018.01)* **C12Q 1/6867** *(2018.01)*
**C12Q 1/6806** *(2018.01)*

(52) Cooperative Patent Classification (CPC):
(C-Sets available)
**C12Q 1/6886; C12Q 1/6806; C12Q 1/6867** (Cont.)

(86) International application number:
**PCT/US2023/063268**

(87) International publication number:
**WO 2023/164650 (31.08.2023 Gazette 2023/35)**

(54) **ORI-C MEDIATED EXTRACHROMOSOMAL DNA AMPLICATION AND SEQUENCING FOR DIAGNOSING, SCREENING AND TREATING DISEASES**

ORI-C-VERMITTELTE EXTRACHROMOSOMALE DNA-AMPLIFIKATION UND -SEQUENZIERUNG ZUR DIAGNOSE, ZUM SCREENING UND ZUR BEHANDLUNG VON KRANKHEITEN

AMPLIFICATION ET SÉQUENÇAGE D'ADN EXTRACHROMOSOMIQUE À MÉDIATION ORI-C POUR LE DIAGNOSTIC, LE CRIBLAGE ET LE TRAITEMENT DE MALADIES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **24.02.2022 US 202263313549 P**

(43) Date of publication of application:
**27.11.2024 Bulletin 2024/48**

(73) Proprietor: **The Jackson Laboratory**
**Bar Harbor, ME 04609 (US)**

(72) Inventors:
• **WEI, Chia-lin**
**Bar Harbor, ME 04609 (US)**
• **NGAN, Chew, Yee**
**Bar Harbor, ME 04609 (US)**

(74) Representative: **Barker Brettell LLP**
**100 Hagley Road**
**Edgbaston**
**Birmingham B16 8QQ (GB)**

(56) References cited:
**WO-A1-2016/026954    WO-A1-2020/210802**
**US-B2- 10 301 672**

• **FENG WEIJIA ET AL: "Targeted removal of mitochondrial DNA from mouse and human extrachromosomal circular DNA with CRISPR-Cas9", COMPUTATIONAL AND STRUCTURAL BIOTECHNOLOGY JOURNAL, vol. 20, 1 January 2022 (2022-01-01), Sweden, pages 3059 - 3067, XP093025452, ISSN: 2001-0370, DOI: 10.1016/ j.csbj.2022.06.028**
• **WANG YUANGAO ET AL: "eccDNAs are apoptotic products with high innate immunostimulatory activity", NATURE, NATURE PUBLISHING GROUP UK, LONDON, vol. 599, no. 7884, 20 October 2021 (2021-10-20), pages 308 - 314, XP037613859, ISSN: 0028-0836, [retrieved on 20211020], DOI: 10.1038/S41586-021-04009-W**

Remarks:
The complete document including Reference Table(s) and the Sequence Listing(s) can be downloaded from the EPO website

EP 4 466 381 B1

(52) Cooperative Patent Classification (CPC): (Cont.)

C-Sets
**C12Q 1/6806, C12Q 2525/307, C12Q 2531/125,
C12Q 2535/122;
C12Q 1/6867, C12Q 2525/307, C12Q 2531/125**

**Description**

**CROSS-REFERENCE TO RELATED APPLICATIONS**

This application claims priority to U.S. Provisional Patent Application No. 63/313,549 filed on February 24, 2022,

**FIELD OF THE DISCLOSURE**

The present invention relates to compositions and methods for amplifying and sequencing circular DNA and, specifically eukaryotic extrachromosomal circular DNA (eccDNA). Specifically, the invention relates to amplifying human extrachromosomal circular DNA for isolating, sequencing, subsequent screening for disease and identifying and providing treatment thereof.

**BACKGROUND OF THE DISCLOSURE**

Extrachromosomal circular DNA (eccDNAs) are circular DNA species found in eukaryote genomes ranging from yeast to humans. Although little is known regarding the biological purposes and function of eccDNAs *in vivo,* it is generally understood that these nucleic acid species carry genetic sequences of protein-coding sequences or repetitive elements with many functions including cell regulation, proliferation, environmental adaptation, genome stability and stimulation of immune responses. It has been postulated that the presence of eccDNAs and their genetic diversity confer fitness advantages. Additionally, cancer studies demonstrate that eccDNAs play a role in driving human tumor malignancy, including metastasis, and drug resistance. Despite these findings in cancerous cells, the specific function of eccDNAs in normal cells remains unknown. The mystery surrounding the role of eccDNA involves multiple factors, including for example lower levels of eccDNA in a cell relative to other forms of DNA, the highly heterogeneous nature of eccDNA itself, and a dearth of known sequences that can used as a basis for further experimentation. Wang et al., 2021, Nature 599: 308-314, discloses the preparation of Illumina sequencing libraries for eccDNA by Tn5-transposon-based tagmentation and sequencing to study the role of eccDNA in apoptosis and cancer. US 10 301 672 discloses the use of a replication of origin sequence present in a circular DNA to amplify the circular DNA. Therefore, there exists a need to efficiently amplify and characterize human eccDNA that currently does not exist in the art.

**SUMMARY OF THE DISCLOSURE**

Described herein are compositions, methods, and kits for amplifying and characterizing human extrachromosomal circular DNAs (eccDNA). In some aspects of this invention are provided methods called OREO-seq (OriC-mediated Extrachromosomal Circles (O)).

In one preferred aspect, OREO-seq based sequencing can specifically amplify eccDNAs from biological samples including extracted genomic DNA, permeabilized nuclei, body fluid (including but not limited to plasma, urine, serum, and cerebrospinal fluid) and convert them to templates for long-read sequencing analysis. OREO-seq uses an in vitro assembled transposome carrying a replication origin sequence from the E coli chromosome (oriC) to specifically tag and exponentially amplify eccDNAs from a eukaryotic cell extract. These methods comprise engineering into eukaryotic DNA a transposon that contains oriC and transposases to catalyze a transposition reaction. Once the transposome (a complete transposon-transposase complex) is introduced to cellular genetic material, it randomly binds and catalyzes a transposition reaction-inserting oriC into both chromosomal DNA as well as eccDNA. However, because eccDNAs are circular by nature and thereby similar to a bacterial genome, exposing the genetic material to the conditions necessary for an isothermal replication cycle reaction causes the eccDNAs that possess the transposed OriC region to replicate while the chromosomal DNA does not.

Additionally, in certain embodiments, treatment with an Exo V nuclease can be used to assist the breakdown of remaining linear products for higher resolution of amplified eccDNA or ecDNA.

Furthermore, in yet another embodiment, the amplification products can be treated with PacI to digest circular background mitochondrial DNA. Mitochondrial DNA digestion permits higher resolution of amplified eccDNA or ecDNA products. These aspects of the inventive methods permit large amounts of eccDNA or ecDNA to be replicated selectively over eukaryotic chromosomal DNA in vitro.

The OriC sequences in the amplified eccDNA can then be used as signatures for targeted capture and subjected for long-read sequencing. Once purified, the eccDNA can be cleaved at engineered predetermined locations by CRISPR/Cas9 or restriction enzymes along the engineered OriC sequences resulting in purified linear strands of eccDNA or ecDNA. Various sequencing methods, such as long-read sequencing, can provide full length eccDNA or ecDNA structure characterization which can assist in elucidating the functions of said DNA products and associated disease product therein.

These and other features, objects, and advantages of the present invention will become better understood from the description that follows. In the description, reference is made to the accompanying drawings, which form a part hereof and in which there is shown by way of illustration, not limitation, embodiments of the invention. The description of preferred embodiments is not intended to limit the invention to cover all modifications, equivalents, and alternatives. Reference should therefore be made to the claims recited herein for interpreting the scope of the invention.

## BRIEF DESCRIPTION OF THE DRAWINGS

FIG. 1 depicts a representative schematic diagram of a chromosomal DNA 100, present in eukaryotic chromosome along with representations of extrachromosomal circular DNA (eccDNA) 101 and extrachromosomal circular DNA (eccDNA) of various sizes 102.

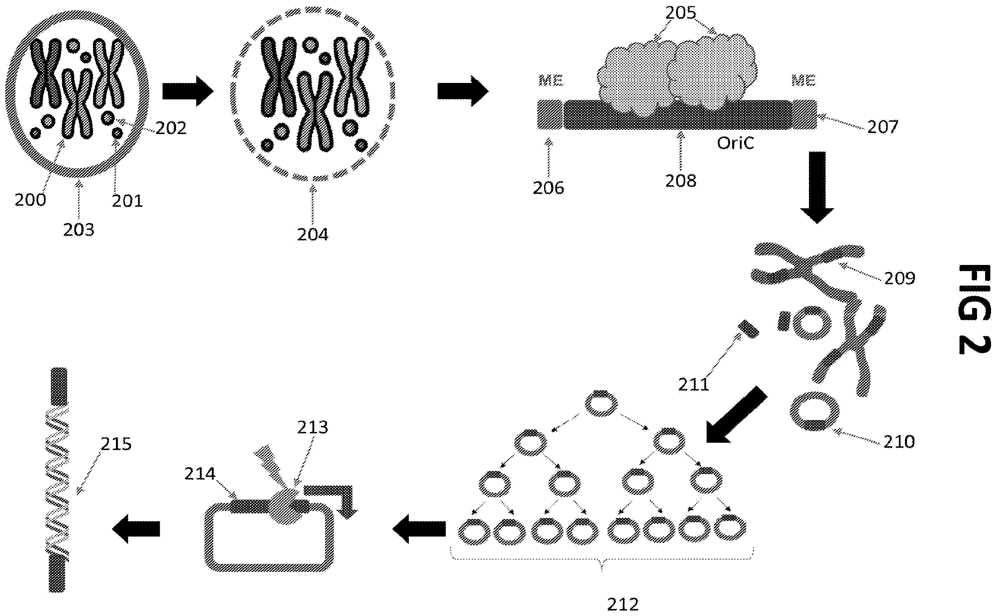

FIG. 2 depicts chromosomal DNA 200, eccDNA 201 and eccDNA of various sizes 202, present within the nucleus 203 of a eukaryotic cell. After the nucleus is lysed 204, the nuclear DNA is treated with a transposase 205, that binds with specificity to an OriC cassette 208, the cassette positioned between a 5 prime terminal 19 base pair mosaic end 206, and a 3 prime terminal 19 base pair mosaic end, 207. The transposition reaction is non-specific to DNA targets and will randomly insert into both chromosomal DNA 209, and eccDNA 201, to form a circular OriC-DNA oligo 210. Some OriC DNA oligos 211, will remain unreacted. When treated with the necessary enzymes, the circular OriC-DNA oligos will be exponentially amplified 212, through an isothermal replication cycle reaction (RCR). The amplified transposed OriC-DNA oligos 214, can be further be treated with a cleaving enzyme 213, resulting in a liner DNA oligo suitable 215, suitable for sequencing.

FIG. 3 depicts one embodiment of the present invention in which a transposon cassette 311, is inserted via an assembly reaction into a portion of eccDNA 301. The transposed eccDNA 310 is then exponentially amplified using a replication cycle reaction 312.

FIG. 4 depicts an agarose gel in which a prokaryotic origin of replication, specifically OriC, was successfully inserted via an assembly reaction into a portion of eccDNA.

FIG. 5 depicts a representative schematic diagram of the transposition reaction, including recruitment of a transposase 505, formation of a fully assembled transposome 516, with an OriC-DNA oligo cassette 511, and catalysis of a transposon-complex-dependent transposition reaction 515 with a piece of ecDNA 501 to form a Circular OriC-DNA oligo 510. FIG 5 further depicts exponential amplification 512.

FIG. 6 depicts an agarose gel in which a prokaryotic origin of replication, specifically, OriC was successfully inserted into a portion of eccDNA and subject to two rounds of amplification.

FIG. 7 depicts an agarose gel in which a prokaryotic origin of replication, was successfully inserted into a portion of eukaryotic eccDNA and amplified, then visualized using SYBR safe DNA gel staining.

FIG. 8 depicts an agarose gel in which a prokaryotic origin of replication, was successfully inserted into a portion of eukaryotic eccDNA from a cancerous cell line and amplified.

FIG. 9 depicts an agarose gel in which a prokaryotic origin of replication, was successfully inserted into a portion of eukaryotic eccDNA from a healthy B-lymphocyte cell line and amplified.

FIG. 10 depicts an agarose gel in which a prokaryotic origin of replication, was successfully inserted into a portion of eukaryotic human eccDNA present in plasma and cerebral spinal fluid and amplified.

FIG. 11 depicts an agarose gel in which a prokaryotic origin of replication, was successfully inserted into a portion of eukaryotic human eccDNA present in human B-lymphocytes and amplified. DNA from the cell sample was then treated with PacI to cleave circular mitochondrial DNA and Exo V was applied to further digest linearized DNA products. As shown, treatment with PacI and Exo V provided higher resolution of amplified products.

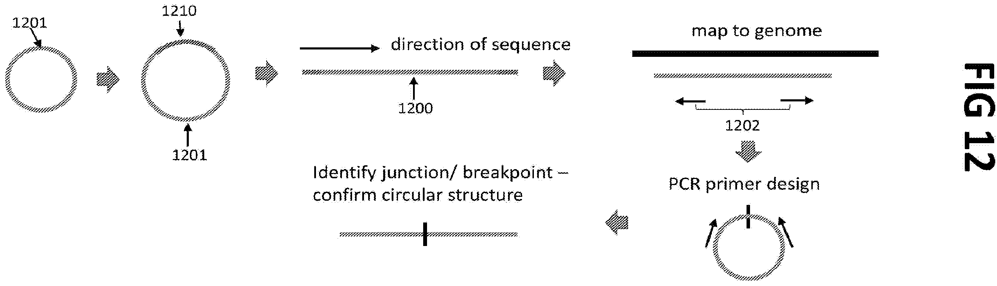

FIG. 12 illustrates a process for determining whether a particular sequence is present in a cell or cellular nucleic acid extract as an eccDNA. An eccDNA molecule 1201, from a cell or cellular nucleic acid extract is converted according to the methods set forth herein to contain an OriC sequence 1210, amplified and linearized to product a fragment having portions of the OriC sequence at each end 1200, and then sequenced. From that sequence a pair of primers 1202 is produced that can be extended away from one another by PCR. Any linear fragment will not be amplified. These primers are then used for PCR amplification of the eccDNA molecule in a sample from a cell or cellular nucleic acid extract. Production of a PCR amplified product indicates that the sequence is present in an eccDNA in the cell or cellular nucleic acid extract.

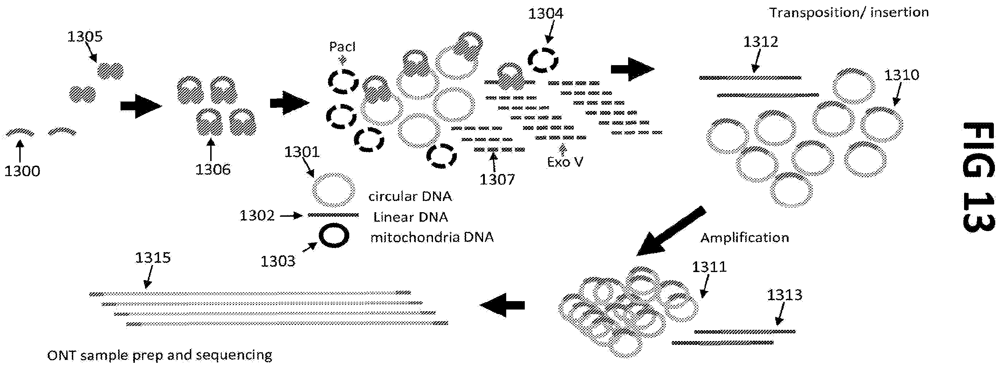

FIG. 13 illustrates the workflow of the method, wherein OriC fragments 1300 generated by amplification with Oreo7 (SEQ ID NO. 3) and Oreo8 (SEQ ID NO. 4) primers and digested with restriction enzyme Srfl are introduced by the action of transposase 1305, into circular DNA species 1301, isolated from a DNA cellular source treated with PacI and Exo V that preferentially digests mitochondrial 1303, and linear genome DNA 1302, respectively. OriC-containing eccDNA species 1310, are amplified 1311, as disclosed herein, linearized, and subject to nucleotide sequencing protocols (e.g., NGS as set forth below). The transposase 1305, forms a transposon complex 1306, with the OriC fragment 1300. Cleaved mitochondrial DNA 1304, and cleaved linear gnome DNA 1307, provide for a higher degree of selectivity for the transposition reaction, as the target Circular DNA species 1301 remain intact. Although there will be non-selective linear genomic transposition reaction products 1312, these products are incapable of undergoing the replication cycle reaction and therefore will not be amplified 1313. The amplified OriC-containing eccDNA species 1311 are then linearized 1315, and prepared for sequencing.

FIG. 14 shows the outcome of these protocols prior to nucleotide sequencing, resulting in three different preparations having a molecular size of about 2kb.

FIG. 15 shows the sequence statistics for all species (top graph) and a specific aligned human sequence, hg38 (bottom graph); the latter had a higher median read length than for all species (1,864bp vs. 1027bp); read lengths for hg38 were less heterogeneous.

FIG. 16 shows alignment of reads to the human genome CHM13 T2T v1.1.

FIG. 17 shows an agarose gel of the pUC plasmid amplified after OriC transposition.

FIG. 18 shows an agarose gel of OriC-mediated amplification on circular and linear DNA, wherein Tn5-Oric treated circular pUC19 is amplified in the presence (lanes 1-3) and absence (lanes 4-6) of lambda phage DNA fragmented by PmeI treatment.

FIG. 19 shows an agarose gel of the amplification of circular DNA from cells transfected with a 5.5kb plasmid. At the highest tested concentrations of DNA from cells not transfected with plasmid DNA a 2.5 kb band was detected which arose from human chromosome 1-specific repetitive DNA

FIG. 20 shows the sequenced band from the 2.5 kb circular DNA detected in FIG. 19. The sequence aligned with human DNA from the T2T (telomere-to-telomere) genomic DNA sequence. The alignment showed that these sequences were from chromosome 1-specific repeats.

FIG. 21 shows an agarose gel of DNA extracted from heart muscle from 6 C57Bl mice (3 males and 3 females) and subjected to OriC-mediated amplification.

FIG. 22 shows atomic force microscope (AFM) images from plasmid-transfected PC3 cells as shown in FIG. 19 and DNA from mouse heart as shown in FIG 21.

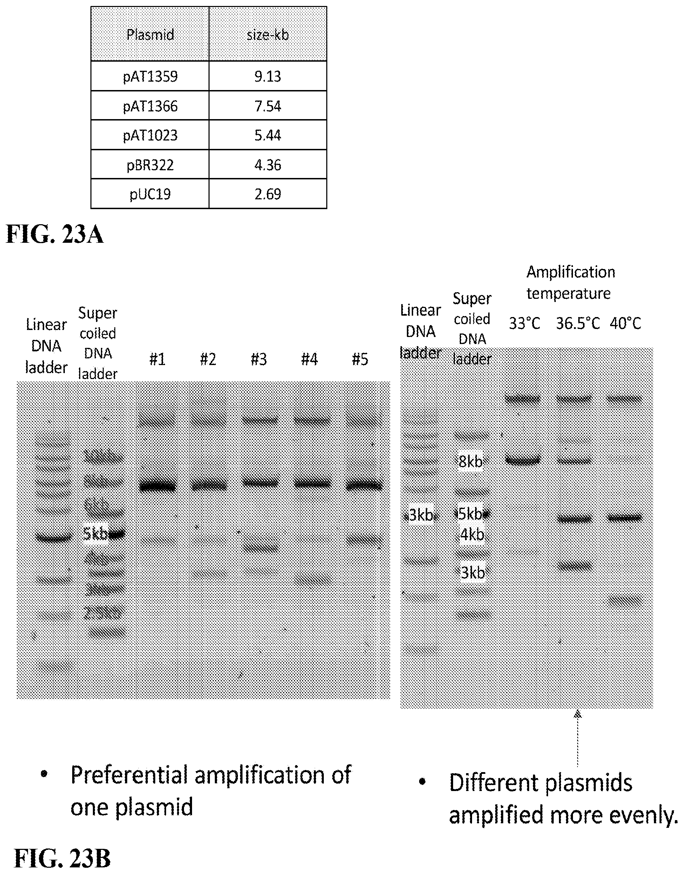

FIG. 23A-FIG 23.B show amplification of plasmid under various conditions. FIG. 23A shows the sizes of the plurality of five plasmids amplified together in a single reaction. FIG. 23B shows an agarose gel of the plasmids amplified together in a single reaction shown in FIG. 23A over various temperatures 33 degrees °C, 36.5 degrees °C, or 40 degrees °C.

FIG. 24 shows an agarose gel of various species of eccDNA detected in each tissue in two mice (#1, #2), wherein the detected species differed between the different tissue in the same mouse and the tissues of the different mice.

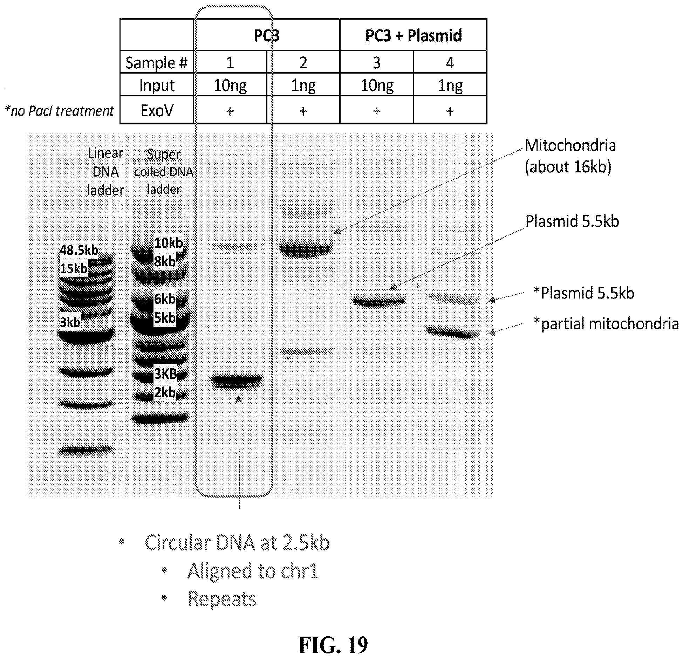

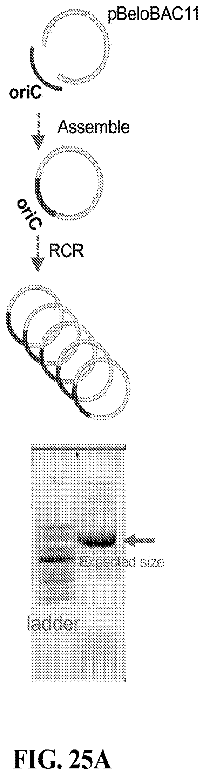

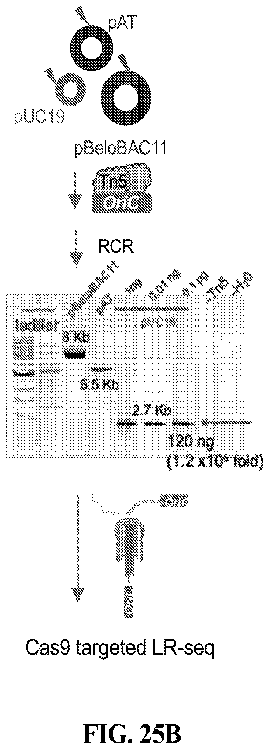

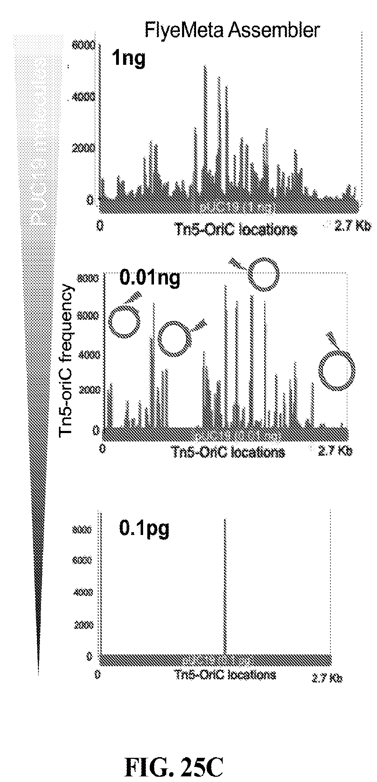

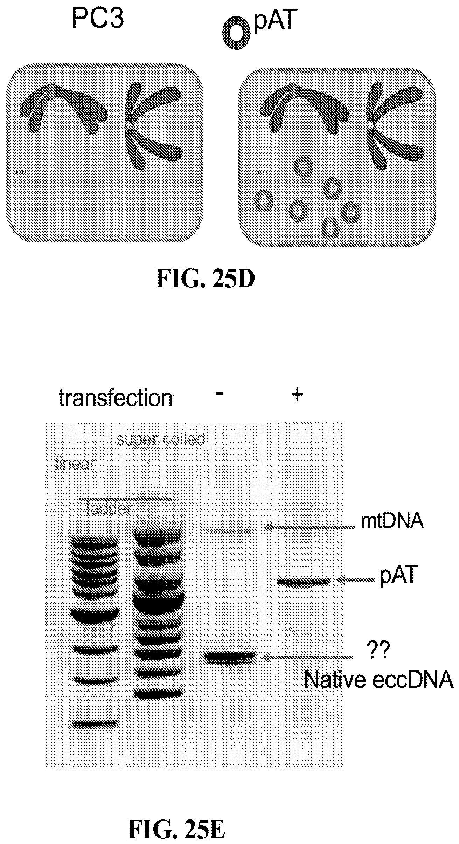

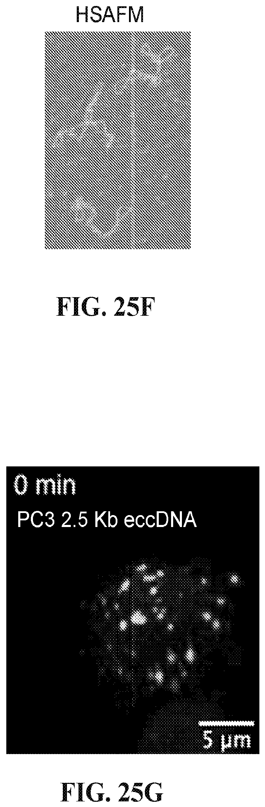

FIG. 25A- FIG. 25G show a summary illustrating the invention disclosed herein. FIG. 25A shows transposase-mediated insertion of OriC into pBeloBAC11, followed by RCR amplification and detection of the amplified circular DNA. FIG. 25B shows transposase-mediated insertion of OriC using pUC19 and pAT plasmids, followed by RCR amplification and detection of the amplified circular DNA. FIG. 25C shows the locations in pUC19 plasmids of OriC insertion using decreasing amounts of Tn5-OriC that resulted in fewer and more specific insertion sites for the origin. FIG. 25D illustrates a diagram of the experimental results shown in FIG. 19. FIG. 25E shows an agarose gel of the experimental results shown in FIG. 19. FIG. 25F shows the presence of circular DNA species detected by atomic force microscopy. FIG. 25G shows a photomicrograph of histochemical staining of the 2.5Kb eccDNA species detected in the PC3 cells of FIG. 19.

DETAILED DESCRIPTION OF THE DISCLOSURE

For the purposes of explicating and understanding the principles of this disclosure, reference is made to embodiments and specific language used to describe the same. The skilled artisan will nevertheless understand that no limitation of the scope of the disclosure is thereby intended, such alteration and further modifications of the disclosure as illustrated herein, being contemplated as would be understood by one skilled in the art to which the disclosure relates.

Definitions

As used herein, articles "a" and "an" are intended to refer to one or to more than one (i.e., at least one) of the grammatical object of the article. By way of example, "an element" means at least one element and can include more than one element.

"About" is used to provide flexibility to a numerical range endpoint by providing that a given value can be "slightly above" or "slightly below" the endpoint without affecting the therapeutically beneficial result. The term "about" in association with a numerical value means that the numerical value can vary by plus or minus 5% or less of the numerical value.

Throughout this specification, unless the context requires otherwise, the word "comprise" and "include" and variations (e.g., "comprises," "comprising," "includes," "including") will be understood to imply the inclusion of a stated component, feature, element, or step or group of components, features, elements, or steps but not the exclusion of any other integer or step or group of integers or steps.

As used herein, "and/or" refers to and encompasses any and all possible combinations of one or more of the associated listed items, as well as the lack of combinations where interpreted in the alternative ("or").

Recitation of ranges of values herein are merely intended to serve as a succinct method of referring individually to each separate value falling within the range, unless otherwise indicated herein. Furthermore, each separate value is incorporated into the specification as if it were individually recited herein. For example, if a range is stated as 1 to 50, it is intended that values such as 2 to 4, 10 to 30, or 1 to 3, etc., are expressly enumerated in this disclosure. These are only examples of what is specifically intended, and all possible combinations of numerical values between and including the lowest value and the highest value enumerated are to be considered to be expressly stated in this disclosure.

Unless otherwise defined, all technical terms used herein have the same meaning as commonly understood by a person of ordinary skill in the art to which this disclosure belongs.

Provided herewith is a more detailed description of the compositions, methods, and kits comprising the invention, which is provided to explain and enhance but not replace or be a substitute for the claims set forth below.

There are multiple names for different types of small circular DNA found in eukaryotes. As discussed above, eccDNA is extrachromosomal circular DNA, typically referring to such extrachromosomal circular DNA found in mammals. By contrast, ecDNA is extrachromosomal DNA typically associated with oncogenesis and tumor proliferation. eccDNA can be either oncogenic, or healthy, but ecDNA is usually associated with cellular disease-states, particularly cancer.

Complete enzymatic replication of plasmids containing the origin of Escherichia coli chromosomal replication has been described extensively in the art. See Funnel et al., 1986, J Biol Chem. 261: 5616-5624. For example, Funnel and colleagues described reconstitution of a complete cycle of oriC plasmid replication in vitro using purified proteins, beginning and ending with supercoiled molecules. However, in the past, such replication resulted in limited products.

A common method for amplifying DNA in vitro is the polymerase chain reaction (PCR). However, PCR is limited to amplifying linear DNA as the proteins involved in the process do not naturally possess the capabilities of replicating circular DNA. In vitro circular DNA amplification has been achieved by a separate mechanism used by phage $\lambda$, namely the rolling circle amplification process (RCA). The amplification product obtained by the RCA method is a linear DNA in which the DNA sequence of one round of circular DNA is repeated and is used exclusively for preparing template DNA for additional replication.

Researchers have identified many unique plasma eccDNAs in human blood plasma, but characterization of such eccDNAs has continued to be elusive. Zhu et al., 2017, Sci Reports 7:10968.

Wang and colleagues have identified that eccDNAs are apoptotic products with high innate immunostimulatory activity. See Wang et al., 2021, Nature 599: 308-317. They showed that in mouse cell lines, eccDNA could be isolated and then purified using ligase-assisted minicircle accumulation (LAMA). Wang and colleagues linearized eccDNA products using sequential treatments of nickase fnCpf1 in addition to S1 nuclease and used nanopore sequencing for preparing eccDNA libraries.

Fakruddin and colleagues discuss loop-mediated isothermal amplification as an alternative to PCR. See Fakruddin et al., 2013, J Pharm Bioallied Scie. 5: 245-252.

Peng and Marians further demonstrated that Escherichia coli rely on topoisomerase IV encoded by parC and parE for separation of the daughter chromosomes in chromosomal replication. Peng & Marians, 1993, Proc. Natl. Acad. Sci. USA 90: 8571-8575.

Additionally, decatenation of the linked daughter chromosomes after replication is an important step in oriC-mediated DNA replication. In the oriC replication system, DNA gyrase-catalyzed decatenation of daughter DNA molecules is very inefficient while topoisomerase III is capable of complete decatenation. Hiasa et al., 1994, J. Biol Chem 269: 2093-2099. In contrast topoisomerase I is incapable of decatenation, and instead, only inhibits DNA synthesis.

Researchers have demonstrated that plasmid DNA templates containing oriC and two Ter sites can be used to study the role of Tus in terminating bidirectional replication. See Hiasa et al., J. Biol Chem 269: 26959-26968. Hiasa and colleagues showed that in the absence of Tus, over-replication required DNA ligase and arose via a template strand-switching mechanism.

Researchers have also identified critical enzymes to accomplish the replication cycle of a circular chromosome absent the formation of a linearized product. See Masayuki et al, 2017, Nucleic Acids Research 45: 11525-11534. Masayuki and colleagues were able to reconstitute the entire replication cycle with fourteen purified enzymes that catalyze initiation at oriC, bidirectional fork progression, Okazaki-fragment maturation, and decatenation of the replicated circular products. Using the replication-cycle reaction (RCR) circular DNA was capable of being replicated exponentially as intact covalently closed molecules.

Finally, Nara and Masayuki have shown that it is possible to amplify whole large plasmids via transposon-mediated oriC insertions in vitro. See Nara & Masayuki, 2021, BioTechniques 71: 1-6. Specifically, Nara and Masayuki demonstrated that by using a replication cycle reaction catalyzed by a hyperactive Tn5 transposase, and an oriC-containing transposon, that circular DNA could be replicated efficiently.

The invention as disclosed and claimed herein will be understood by the skilled worker in the art in the context of the understanding in the art of eccDNA, ecDNA, and the ability to replicate bacterial and plasmid DNA using species-specific origins of replication and bacterial enzymes that facilitate such replication.

In one embodiment, the invention comprises a composition further comprising a plurality of eukaryotic extrachromosomal circular DNA (eccDNA) or eukaryotic circular DNA isolated from a cancerous cell, (ecDNA), wherein the circular DNA species have been modified by insertion of a prokaryotic origin of replication.

The composition of circular DNA can be further modified to contain a plurality of circular DNA species that are capable of being amplified by replication in vitro mediated by a prokaryotic origin of replication.

The origin of replication can be a bacterial origin of replication, in particular E. coli OriC. Specifically, the origin of replication can be imbedded into a transposable cassette. The transposable cassette can comprise the following nucleic acid sequence:

5'-CTGTCTCTTATACACATCTgaagatccggcagaagaatggagtatgttgtaactaaagataacttcgtataatgtatgct atacgaagttatacagatc gtgcgatctactgtggataactctgtcaggaagcttggatcaaccggtagttatccaaagaacaactgttgt tcagtttttgagttgtgtataacccctcattctgatcccag cttatacggtccaggatcaccgatcattcacagttaatgatcctttccaggtt gttgatcttaaaagccggatccttgttatccacagggcagtgcgatcctaataagagat cacaatagaacagatctctaaataaaatagatc ttcttttaatacccaggatccatctatgtcgggtgcggagaaagaggtaatgaaatggctttagttacaacatactc aggtctttctcaagc cgacAGATGTGTATAAGAGACAG-3' (SEQ ID NO:7). Other origins of replication can be used as well.

The plurality of circular DNA is produced by enzymatic amplification, in particular isothermal replication cycle reaction (RCR) amplification in vitro. RCR can be carried out by various protocols known to one of ordinary skill in the art. For example, a technique as described by Masayuki and colleagues, Masayuki et al., 2017, Nucleic Acids Research 45: 11525-11534, can be used to amplify eccDNA and ecDNA.

Alternatively, RCR can be carried out using a commercial kit such as the OriCiro® Cell-Free Cloning System provided by OriCirco genomics,

In some embodiments of the invention, the cells from which the eccDNA and ecDNA are isolated are eukaryotic cells. In yet another preferred embodiment, the cells from which the eccDNA or ecDNA are isolated are mammalian cells. In an exemplary embodiment of the present invention, the cells from which the eccDNA or ecDNA are isolated are human cells. Circular DNA obtained from prokaryotic cells can too be utilized in the present invention.

In one embodiment, next generation DNA sequencing, for example, ultra-long read sequencing provided by Oxford Nanopore Technologies can be used to sequence the amplified and isolated eccDNA and ecDNA. See Kazuo et al., 2020, Front. Cell. Infect. Infect. Microbiol. 10: 11. Alternatively, eccDNA or ecDNA can be sheared into 100-300 bp by sonication. The sheared DNA can then be subjected to ThruPLEX® DNA-seq kit provided by Rubicon Genomics. Sequencing can be further assisted with software such as that provided by DNASTAR.

In another embodiment, the eukaryotic circular DNA can be linearized in preparation for sequencing with a CRISPR-associated protein such as Cas9 and an associated guide RNA (crRNA). Other CRISPR associated proteins can be utilized as well such as Cpf1, dCas, dCas9, Mutated Cpf1-dCpf1, and associated crRNAs and trcrRNAs. In yet another embodiment, the eukaryotic circular DNA can be linearized with a restriction enzyme that would be known to one of ordinary skill in the art. In some exemplary embodiments, a restriction site can be engineered into the oriC transposition primers that will later be inserted into the circular DNA sets. In other exemplary embodiments, the restriction site can correspond to SrfI. In other exemplary embodiments, the restriction site can correspond to PmeI.

In some embodiments, the eukaryotic DNA can be cancerous DNA, (ecDNA). ecDNA can be attributable to a plurality of different types of cancers. In other embodiments the circular eukaryotic DNA is not an oncogenic eccDNA but can code for other disease processes.

In some embodiments, the circular DNA is isolated from a tissue. Tissues include but are not limited to neural tissue, thyroid papillary or medullary tissues, esophageal tissues, squamous cell epithelium from the mouth, tongue, nasopharynx, vagina, cervix, or rectum, tissue from the dermis, venous tissue, myocytes, cardiomyocytes, tissue isolated from the thymus, blood, lymphatic fluid, bone, marrow, connective tissues such as fibrocartilage, hyaline cartilage, and other proteoglycan matrix like tissues, smooth muscle tissues such as those isolated from the stomach, or other tissues such as those present in the duodenum, the small intestine, the large intestine, the colon, the prostate, the testes, the ovaries, the cervix or the uterus. Additionally, tissues isolated from the liver, pancreas, gallbladder, lungs, bladder, breasts, mammillary glands, corneas, or sclera can be suitable sources for isolating circular DNA.

Additionally, in yet other embodiments, the tissue is isolated from a tumor or biopsy thereof. The tissue can comprise cancerous cells isolated from blood of a patient with leukemia or another associated cancer of the blood. In other embodiments, the tissue can comprise cancerous cells isolated from the blood of a patient with a lymphoma or other associated cancer of the lymphatic system.

In yet another embodiment, the circular DNA can be isolated from a biofluid. A biofluid can be any fluid secreted or produced from the body during normal physiologic processes as well as disease processes. Biofluids include but are not limited to sweat, blood, plasma, urine, lymph, semen, mucous, tears, colostrum, cerebral spinal fluid, amniotic fluid, bile, chime, puss, sebum, glandular excretions, and waste products such as excrement or feces.

EP 4 466 381 B1

Some embodiments of the invention comprise a kit comprising nucleic acid encoding a prokaryotic origin of replication, protein components specific for the prokaryotic origin of replication and capable of replicating DNA comprising said prokaryotic origin of replication, and a transposase capable of inserting the prokaryotic origin of replication into human-derived eccDNA, and associated reaction buffers and reagents. The prokaryotic origin of replication can be OriC. Alternatively, other prokaryotic origins of replication can be included in the transposon. The protein components specific for the prokaryotic origin of replication, capable of replicating the DNA, comprise: SSB, IHF, DnaG, DnaN, PolIII, DnaB, RNaseH, Ligase, PolI, GyrA, GyrB, Topo IV, Topo III, and RecQ. As set forth herein, SSB indicates SSB derived from *E. coli,* IHF indicates a combination of IhfA and IhfB derived from *E. coli,* DnaG indicates DnaG derived from *E. coli,* DnaN indicates DnaN derived from *E. coli,* PolIII indicates DNA polymerase III complex consisting of a combination of DnaX, HolA, HolB, HolC, HolD, DnaE, DnaQ, and HolE, DnaB indicates DnaB derived from *E. coli,* DnaC indicates DnaC derived from *E. coli,* DnaA indicates RNaseH derived from *E. coli,* Ligase indicates DNA ligase derived from *E. coli,* PolI indicates DNA polymerase I derived from *E. coli,* GyrA indicates GyrA derived from *E. coli,* GyrB indicates GyrB derived from *E. coli,* Topo IV indicates a combination of ParC and ParE derived from *E. coli,* Topo III indicates topoisomerase III derived from *E. coli,* and RecQ indicates RecQ derived from *E. coli.*

SSB can be prepared by purifying an E. coli strain expressing SSB by steps that include ammonium sulfate precipitation and ion-exchange column chromatography.

IHF can be prepared by purifying an E. coli strain coexpressing IhfA and IhfB by steps that include ammonium sulfate precipitation and affinity column chromatography.

DnaG can be prepared by purifying an E. coli strain expressing DnaG by steps that include ammonium sulfate precipitation and anion-exchange column chromatography and gel filtration column chromatography.

DnaN can be prepared by purifying an E. coli strain expressing DnaN by steps that include ammonium sulfate precipitation and anion-exchange column chromatography.

PolIII can be prepared by purifying an E. coli strain coexpressing DnaX, HolA, HolB, HolC, HolD, DnaE, DnaQ, and HolE by steps that include ammonium sulfate precipitation, affinity column chromatography and gel filtration column chromatography.

DnaB and DnaC can be prepared by purifying an E. coli strain coexpressing DnaB and DnaC by steps that include ammonium sulfate precipitation, affinity column chromatography and gel filtration column chromatography.

DnaA can be prepared by purifying an E. coli strain expressing DnaA by steps that include ammonium sulfate precipitation, dialysis precipitation, and gel filtration column chromatography.

GyrA and GyrB can be prepared by purifying a mixture of an E. coli strain expressing GyrA and an E. coli strain expressing GyrB by steps that include ammonium sulfate precipitation, affinity column chromatography and gel filtration column chromatography.

Topo IV can be prepared by purifying a mixture of an E. coli strain expressing ParC and an E. coli strain expressing ParE by steps that include ammonium sulfate precipitation, affinity column chromatography and gel filtration column chromatography.

Topo III can be prepared by purifying an E. coli strain expressing Topo III by steps that include ammonium sulfate precipitation, and affinity column chromatography.

RecQ can be prepared by purifying an E. coli strain expressing RecQ by steps that include ammonium sulfate precipitation, affinity column chromatography and gel filtration column chromatography.

Commercially available enzymes derived from E. coli can be used for RNaseH, Ligase and PolI, for example those provided by Takara Bio Inc. In the use of these enzymes as set forth in instructions provided by the manufacturer and as further set forth in U.S. Patent No. 10,301,672 to Su'etsugu et al. at col. 13, In 32-50, a 9.6 kb circular DNA can serve as a template having a replication origin sequence oriC and a portion coding for kanamycin resistance. Alternatively, an 80 kb long circular DNA having a replication origin sequence oriC and a portion encoding a kanamycin resistance gene can be used as a template. In yet another alternative, the template can comprise a 200 kb long chain circular DNA having a replication origin sequence oriC and encoding a kanamycin resistance gene. DNA cyclized by in vitro ligation can also serve as a template.

The template long chain circular DNAs can be prepared by in vivo recombination in E. coli cells. Specifically, an in vivo recombination can be performed using E. coli expressing a recombination protein group of a λ phage to prepare circular DNA of a desired length that would also include a cassette coding for kanamycin resistance and a region in the E. coli chromosome that includes oriC.

In a preferred embodiment, the kit comprises a hyperactive Tn5 transposase, and an oriC-containing transposon, that catalyze a transposition reaction into target eccDNA and ecDNA.

In yet another embodiment, the kit can further comprise a purified preparation of CRISPR associated proteins (Cas) and include a corresponding guide mRNA or trcrRNA. The Cas protein can comprise Cas9, dCas, mutated Cas9-nickase, Cpf1, mutated Cpf1-dCpf1, C2c2-Cas13a-mutants, other Cas proteins, and other mutated Cas varieties.

In yet another embodiment, the invention provides methods for producing a plurality of extrachromosomal circular DNA (eccDNA) or (ecDNA) isolated from a eukaryotic cell comprising the steps of (1) isolating cellular DNA comprising circular DNA from the cell; (2) modifying the circular DNA comprising eccDNA or ecDNA by inserting the prokaryotic origin of replication into the isolated circular DNA; (3) amplifying in vitro the modified circular DNA comprising eccDNA or ecDNA; and (4) isolating the amplified eccDNA or ecDNA. The method can further comprise inserting a prokaryotic origin of replication into the isolated circular DNA by a transposase.

In yet another embodiment of the invention, the method can utilize a prokaryotic origin of replication such as E. coli's OriC.

In another preferred embodiment of a method of the invention, the method can utilize a transposon that is assembled with a set of linear DNA oligonucleotide primers. The linear DNA oligonucleotide primers can comprise SEQ ID: 1 and SEQ ID: 2. Alternatively, the linear DNA oligonucleotide primers can comprise SEQ ID: 3 and SEQ ID: 4. In yet another embodiment, the linear DNA oligonucleotide primers can comprise SEQ ID: 5 and SEQ: ID: 6.

TABLE 1.

| SEQ ID: No. | Nucleic Acid Sequence 5'-3' |
| --- | --- |
| SEQ ID: 1 | CTGTCTCTTATACACATCTCTGCTCTGATGCCGCATAG |
| SEQ ID: 2 | CTGTCTCTTATACACATCTGTGTCGGGGCTGGCTTAAC |
| SEQ ID: 3 | CTGTCTCTTATACACATCTGCCCGGGCCTGCTCTGATGCCGCATAG |
| SEQ ID: 4 | CTGTCTCTTATACACATCTGCCCGGGCGTGTCGGGGCTGGCTTAAC |
| SEQ ID: 5 | CTGTCTCTTATACACATCTGTTTAAACCTGCTCTGATGCCGCATAG |
| SEQ ID: 6 | CTGTCTCTTATACACATCTGTTTAAACGTGTCGGGGCTGGCTTAAC |

SEQ ID: 1 in Table 1 above corresponds to a 19 base pair mosaic end used as a primer to synthesize DNA templates of a cassette that contains a prokaryotic transposable origin of replication. In a preferred embodiment, that transposable prokaryotic origin of replication is OriC.

SEQ ID: 2 in Table 1 above corresponds to a 19 base pair mosaic end used as a primer to synthesize DNA templates of a cassette that contains a prokaryotic transposable origin of replication. In a preferred embodiment, that transposable prokaryotic origin of replication is OriC.

SEQ ID: 3 in Table 1 above corresponds to a 19 base pair mosaic end used as a primer to synthesize DNA templates of a cassette that contains a prokaryotic transposable origin of replication that also contains a restriction site. In SEQ ID: 3, that restriction site is SrfI.

SEQ ID: 4 in Table 1 above corresponds to a 19 base pair mosaic end used as a primer to synthesize DNA templates of a cassette that contains a prokaryotic transposable origin of replication that also contains a restriction site. In SEQ ID: 4, that restriction site is SrfI.

SEQ ID: 5 in Table 1 above corresponds to a 19 base pair mosaic end used as a primer to synthesize DNA templates of a cassette that contains a prokaryotic transposable origin of replication that also contains a restriction site. In SEQ ID: 5, that restriction site is PmeI.

SEQ ID: 6 in Table 1 above corresponds to a 19 base pair mosaic end used as a primer to synthesize DNA templates of a cassette that contains a prokaryotic transposable origin of replication that also contains a restriction site. In SEQ ID: 6, that restriction site is PmeI.

In another preferred embodiment of a method of the invention, the method can utilize an isothermal replication cycle reaction (RCR) amplification in vitro to amplify the plurality of eccDNA or ecDNA.

In one embodiment of methods of the invention, the method comprises producing a plurality of extrachromosomal circular DNA (eccDNA) or (ecDNA) isolated from a eukaryotic cell comprising the steps of (1) isolating circular DNA comprising eccDNA or ecDNA from the cell; (2) modifying the circular DNA comprising eccDNA or ecDNA by inserting a prokaryotic origin of replication into the isolated circular DNA; (3) applying an Exo V endonuclease to remove linearized non-circular DNA; (4) applying PacI to linearize the circular mitochondrial DNA; (5) amplifying in vitro the modified circular DNA comprising eccDNA or ecDNA; and (6) isolating the amplified eccDNA or ecDNA. In the described method, the circular DNA comprising eccDNA or ecDNA is human. Additionally, the human eccDNA or ecDNA is isolated from a tissue. The tissue can be a tumor or biopsy thereof. The human eccDNA or ecDNA can also be isolated from a biofluid. As described above, the biofluid can be blood or cerebral spinal fluid (CSF).

In yet another embodiment, the invention provides methods for identifying a nucleic acid sequence of eukaryotic extrachromosomal circular DNA (eccDNA) or (ecDNA) and performing nucleic acid sequencing on the amplified DNA products. The eukaryotic extrachromosomal circular DNA (eccDNA) or (ecDNA) can be isolated from a human cell, tissue, or biofluid. Additionally, the human cell, tissue, or biofluid can be in either a normal physiological state, or alternatively, in a diseased state.

In an exemplary embodiment of a method of the present invention, eukaryotic extrachromosomal circular DNA (eccDNA) or (ecDNA) that has been amplified can be linearized with Cas9 or a restriction enzyme before sequencing, and the sequencing of the amplified DNA product can assist a medical professional or patient to determine whether the patient carries extrachromosomal circular DNA (eccDNA) or (ecDNA) from a cell, tissue, or biofluid sample indicative of a disease state therein. The method of sequencing can be nanopore long-read sequencing. In yet another embodiment the sequencing can serve to screen a cancer patient having metastatic disease for treatment options based on the genetic composition of the patient's diseased sample of ecDNA.

In a particular exemplary embodiment of the methods of the invention, the invention provides a method for identifying a cancer patient having metastatic disease for treatment comprising the steps of (1) isolating circular DNA (ecDNA) from the patient; (2) modifying the ecDNA by inserting a prokaryotic origin of replication into the isolated circular DNA via a transposition reaction; (3) applying an Exo V endonuclease to remove linearized non-circular DNA; (4) applying PacI to linearize the circularized mitochondrial DNA; (5) amplifying in vitro the modified circular DNA comprising ecDNA; (6) purifying the amplified ecDNA; (7) linearizing the amplified ecDNA with a Cas9 enzyme or restriction enzyme; (8) characterizing the amplified product of step 6 using a nanopore long-read sequencing technique; and (9) using the characterization of step 7 to of identify a cancer patient having metastatic disease.

Various exemplary embodiments of compositions and methods according to this invention are now described in the following non-limiting Examples.

## DETAILED DESCRIPTION OF THE DRAWINGS

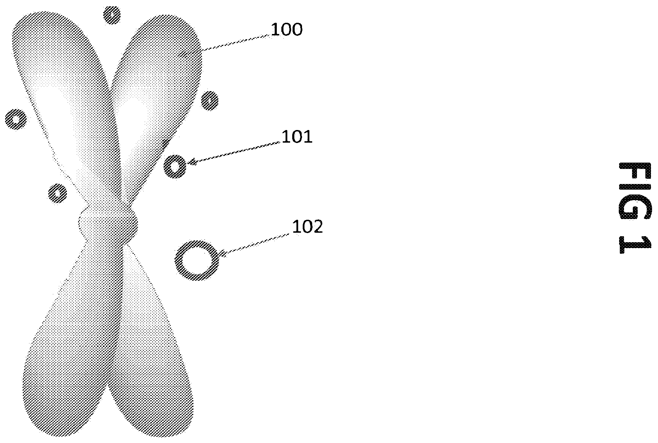

FIG. 1 depicts a representative schematic diagram of a chromosomal DNA 100, present in eukaryotic chromosome along with representations of extrachromosomal circular DNA (eccDNA) 101 and extrachromosomal circular DNA (eccDNA) of various sizes 102.

FIG. 2 depicts chromosomal DNA 200, eccDNA 201 and eccDNA of various sizes 202, present within the nucleus 203, of a eukaryotic cell. After the nucleus is lysed 204, the nuclear DNA is treated with a transposase 205, that binds with specificity to an OriC cassette 208, the cassette positioned between a 5 prime terminal 19 base pair mosaic end 206, and a 3 prime terminal 19 base pair mosaic end, 207. The transposition reaction is non-specific to DNA targets and will randomly insert into both chromosomal DNA 209, and eccDNA 201, to form a circular OriC-DNA oligo 210. Some OriC DNA oligos 211, will remain unreacted. When treated with the necessary enzymes, the circular OriC-DNA oligos will be exponentially amplified 212, through an isothermal replication cycle reaction (RCR). The amplified transposed OriC-DNA oligos 214, can be further be treated with a cleaving enzyme 213, resulting in a liner DNA oligo suitable 215, suitable for sequencing.

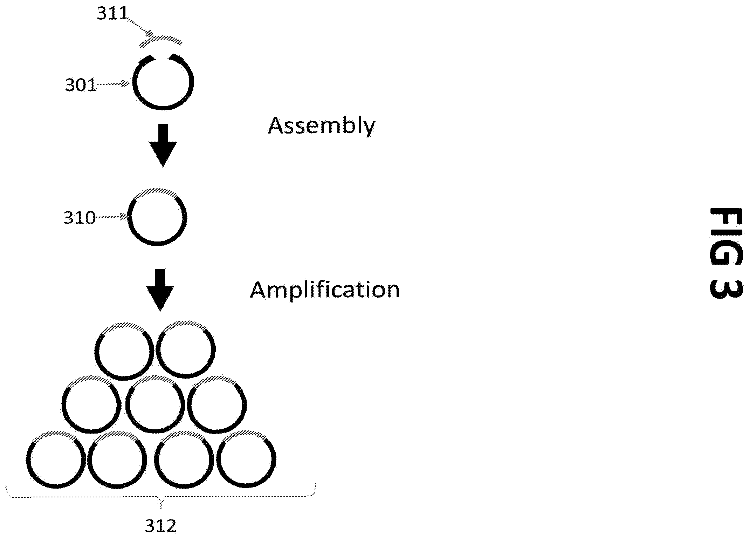

FIG. 3 depicts one embodiment of the present invention in which a transposon cassette 311, is inserted via an assembly reaction into a portion of eccDNA 301. The transposed eccDNA 310 is then exponentially amplified using a replication cycle reaction 312.

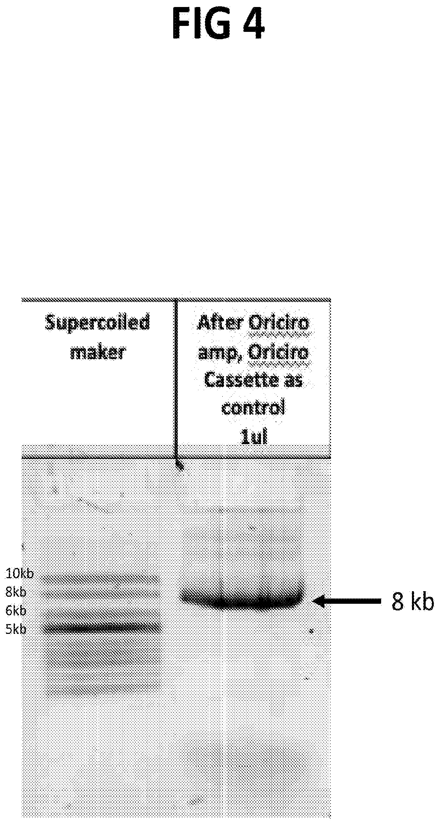

FIG. 4 depicts an agarose gel in which a prokaryotic origin of replication, specifically OriC, was successfully inserted via an assembly reaction into a portion of eccDNA.

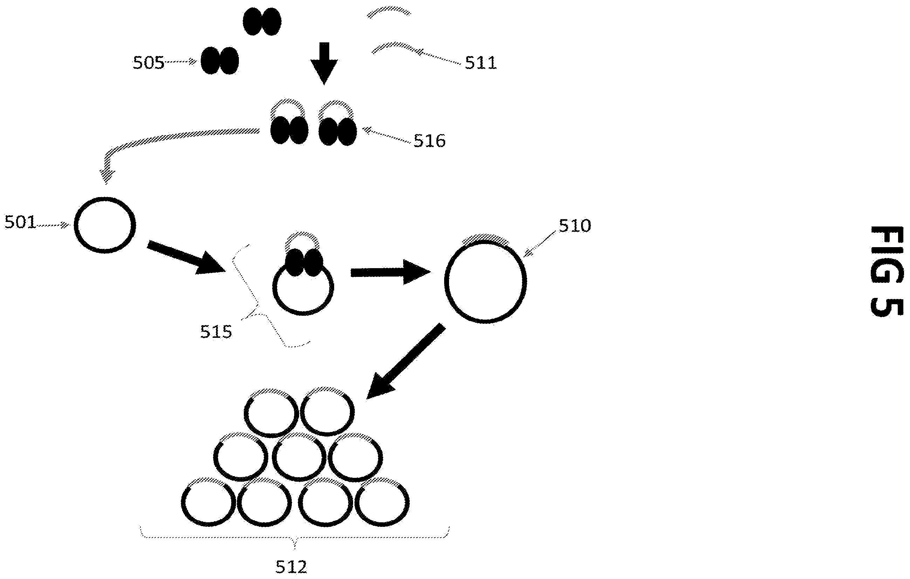

FIG. 5 depicts a representative schematic diagram of the transposition reaction, including recruitment of a transposase 505, formation of a fully assembled transposome 516, with an OriC-DNA oligo cassette 511, and catalysis of a transposon-complex-dependent transposition reaction 515 with a piece of ecDNA 501 to form a Circular OriC-DNA oligo 510. FIG 5 further depicts exponential amplification 512.

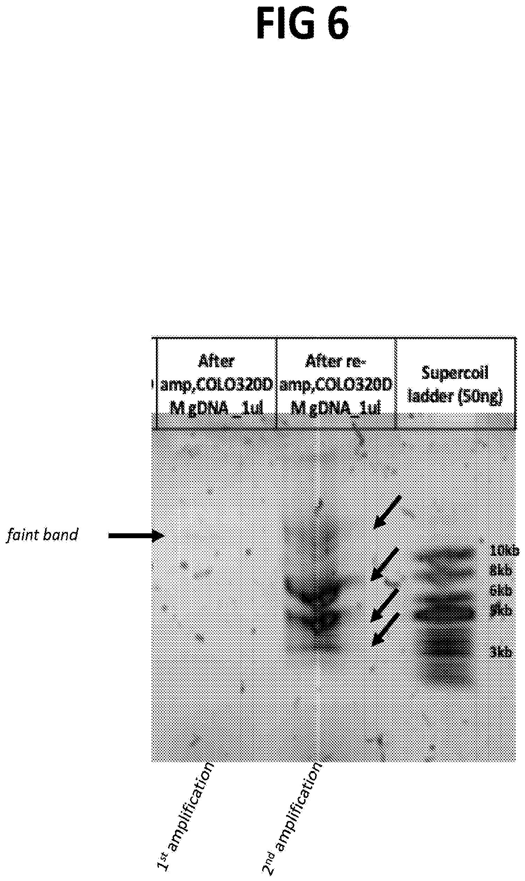

FIG. 6 depicts an agarose gel in which a prokaryotic origin of replication, specifically, OriC was successfully inserted into a portion of eccDNA and subject to two rounds of amplification.

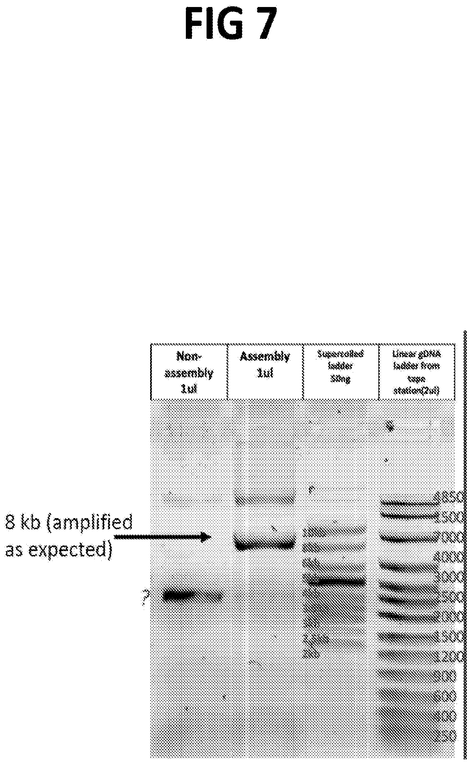

FIG. 7 depicts an agarose gel in which a prokaryotic origin of replication, was successfully inserted into a portion of eukaryotic eccDNA and amplified, then visualized using SYBR safe DNA gel staining.

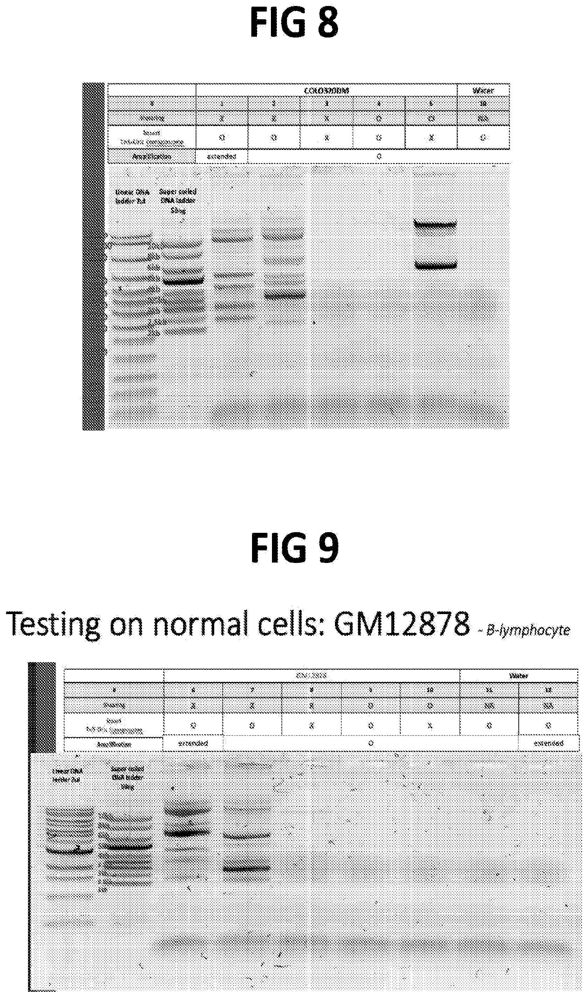

FIG. 8 depicts an agarose gel in which a prokaryotic origin of replication, was successfully inserted into a portion of eukaryotic eccDNA from a cancerous cell line and amplified.

FIG. 9 depicts an agarose gel in which a prokaryotic origin of replication, was successfully inserted into a portion of eukaryotic eccDNA from a healthy B-lymphocyte cell line and amplified.

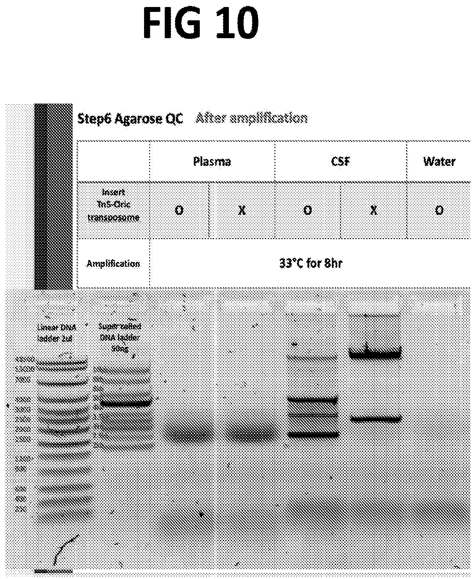

FIG. 10 depicts an agarose gel in which a prokaryotic origin of replication, was successfully inserted into a portion of eukaryotic human eccDNA present in plasma and cerebral spinal fluid and amplified.

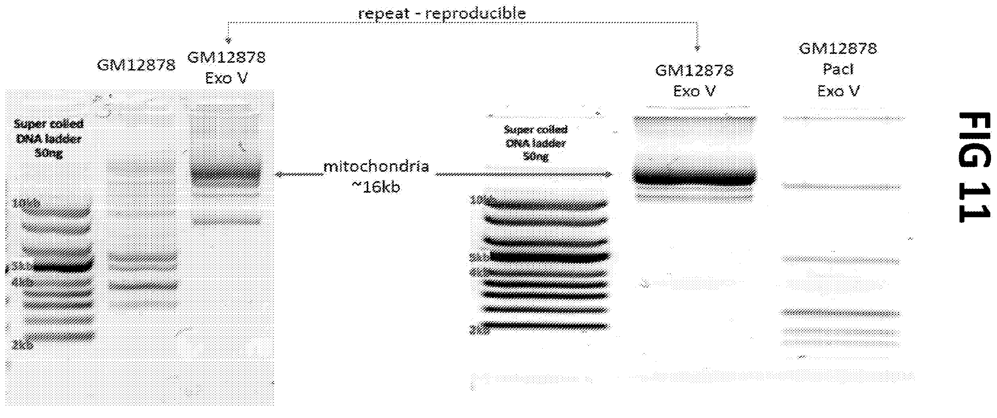

FIG. 11 depicts an agarose gel in which a prokaryotic origin of replication, was successfully inserted into a portion of eukaryotic human eccDNA present in human B-lymphocytes and amplified. DNA from the cell sample was then treated with PacI to cleave circular mitochondrial DNA and Exo V was applied to further digest linearized DNA products. As shown, treatment with PacI and Exo V provided higher resolution of amplified products.

FIG. 12 illustrates a process for determining whether a particular sequence is present in a cell or cellular nucleic acid extract as an eccDNA. An eccDNA molecule 1201, from a cell or cellular nucleic acid extract is converted according to the methods set forth herein to contain an OriC sequence 1210, amplified and linearized to product a fragment having portions of the OriC sequence at each end 1200, and then sequenced. From that sequence a pair of primers 1202 is produced that can be extended away from one another by PCR. Any linear fragment will not be amplified. These primers are then used for PCR amplification of the eccDNA molecule in a sample from a cell or cellular nucleic acid extract. Production of a PCR amplified product indicates that the sequence is present in an eccDNA in the cell or cellular nucleic acid extract.

FIG. 13-16 illustrate efficient sequencing of eccDNA using the methods of the invention and identifying the source of one such sequence in the human genome.

FIG. 13 illustrates the workflow of the method, wherein OriC fragments 1300 generated by amplification with Oreo7 (SEQ ID NO. 3) and Oreo8 (SEQ ID NO. 4) primers and digested with restriction enzyme SrfI are introduced by the action of transposase 1305, into circular DNA species 1301, isolated from a DNA cellular source treated with PacI and Exo V that preferentially digests mitochondrial 1303, and linear genome DNA 1302, respectively. OriC-containing eccDNA species 1310, are amplified 1311, as disclosed herein, linearized, and subject to nucleotide sequencing protocols (e.g., NGS as set forth below). The transposase 1305, forms a transposon complex 1306, with the OriC fragment 1300. Cleaved mitochondrial DNA 1304, and cleaved linear gnome DNA 1307, provide for a higher degree of selectivity for the transposition reaction, as the target Circular DNA species 1301 remain intact. Although there will be non-selective linear genomic transposition reaction products 1312, these products are incapable of undergoing the replication cycle reaction and therefore will not be amplified 1313. The amplified OriC-containing eccDNA species 1311 are then linearized 1315 and prepared for sequencing.

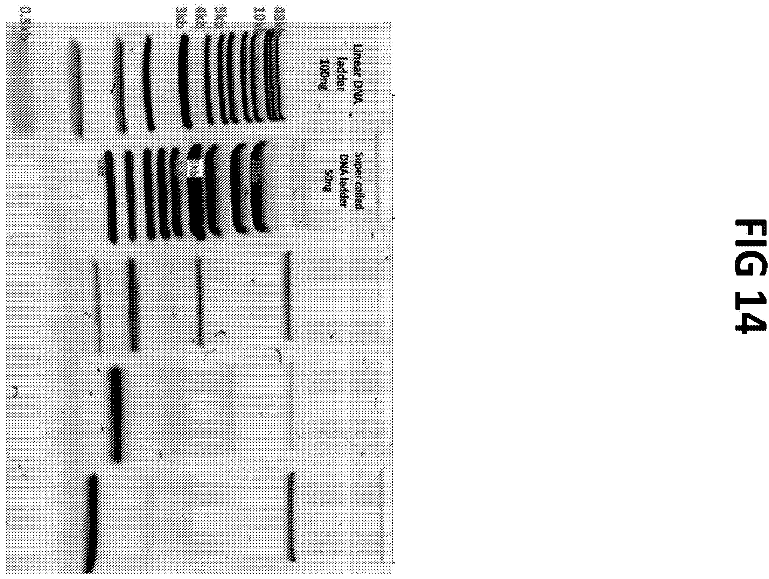

FIG. 14 shows the outcome of these protocols prior to nucleotide sequencing, resulting in three different preparations having a molecular size of about 2kb.

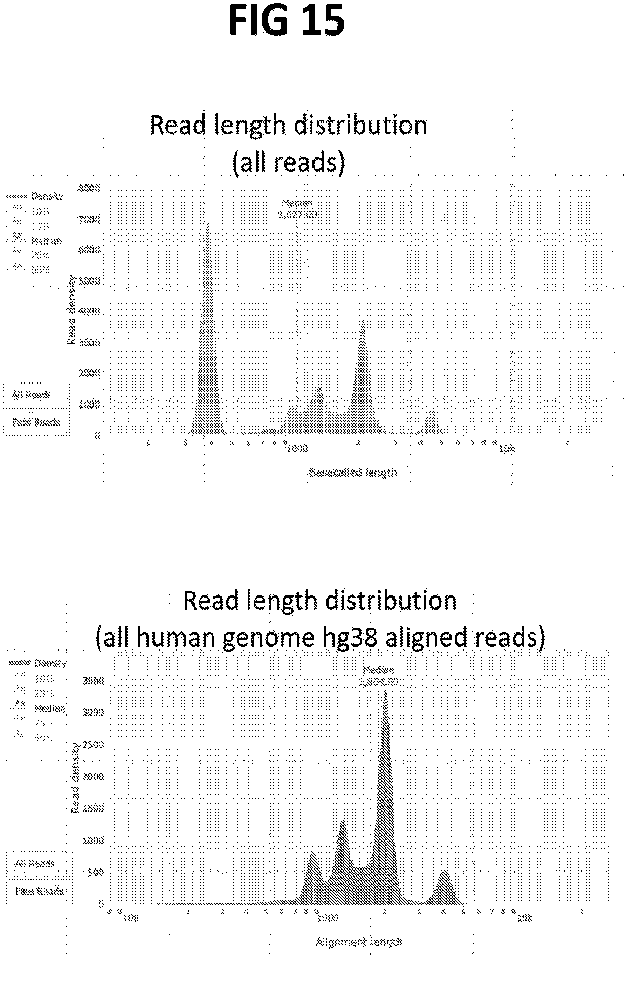

FIG. 15 shows the sequence statistics for all species (top graph) and a specific aligned human sequence, hg38 (bottom graph); the latter had a higher median read length than for all species (1,864bp vs. 1027bp); read lengths for hg38 were less heterogeneous.

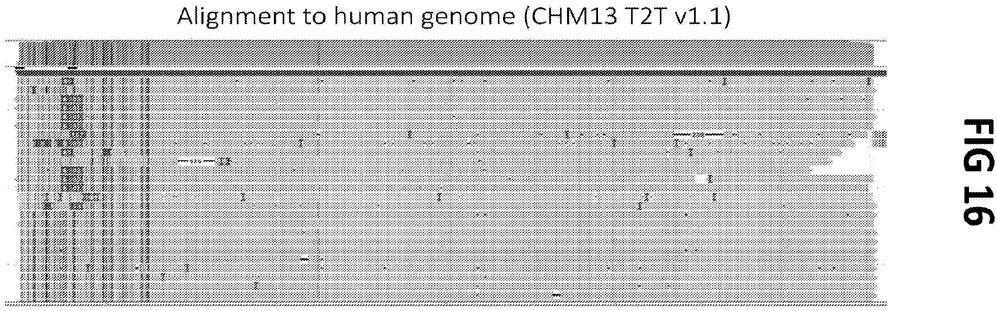

FIG. 16 shows alignment of reads to the human genome CHM13 T2T v1.1.

## EXAMPLES

To generate an OriC containing transposome, OriC DNA oligos were first amplified using primer pairs SEQ ID:1 and SEQ ID:2 listed in Table 1 to introduce 19-bp mosaic ends to the OriC cassette. The OriC cassette was obtained from an OriCiro Cell-Free Cloning System Kit (OriCiro Genomics). Additional OriC DNA oligos aside from SEQ ID:1 and SEQ ID:2 can be used. For example, SEQ ID:3, SEQ ID:4, SEQ ID:5, SEQ ID:6 can also be used in the transposition reaction. SEQ ID:3 through SEQ ID:6 were engineered with restriction sites embedded within the DNA oligo to allow linearization of amplified circular products for long read sequencing treatment after transposition and amplification.

The engineered OriC DNA oligos were then amplified using a polymerase chain reaction (PCR). PCR can be performed by a plurality of protocols that would be known to one of ordinary skill in the art. Specifically, PCR was performed using a KAPA HiFi HotStart ReadyMix kit and protocol (#7958927001, Roche).

The amplified OriC DNA oligo products were then purified. Purification can be accomplished by a plurality of protocols that would be known to one of ordinary skill in the art. Specifically, the OriC DNA oligos were purified using a SPRI bead system (Beckman Coulter).

The transposition reaction was initiated using the following protocol, OriC DNA oligos (2.36pmol) were loaded with a 1$\mu$l aliquot solution of hyperactive Tn5 transposase, Diagenode Tagmentase (C01070010, Diagenode), and incubated at 30°C for 30 min in a thermocycler. Additional OriC DNA oligos aside from SEQ ID:1 and SEQ ID:2 can be used. For example, SEQ ID:3, SEQ ID:4, SEQ ID:5, SEQ ID:6 can also be used in the transposition reaction.

A replication cycle reaction can be prepared using general protocols known by one of ordinary skill in the art. For example, the following protocol by Masayuki et al, Nucleic Acid Res 45(20), 11525-11534 (2017), is generally sufficient to accomplish RCR. As detailed by Masayuki, a 5 × replication-cycle reaction (RCR) buffer (100 mM Tris-HCl pH8.0, 750 mM potassium glutamate, 50 mM ammonium sulfate, 50 mM Mg(OAc)2, 40 mM dithiothreitol (DTT), 20 mM creatine phosphate, 5 mM each NTP, 0.5 mM each dNTP, 0.25 mg/ml yeast tRNA, and 1.25 mM NAD+) and a 4 × Enzyme mix (2 mg/ml bovine serum albumin, 0.08 mg/ml creatine kinase, 0.1 mM adenosine triphosphate (ATP), 1.6 M SSB4, 160 nM IHF2, 1.6 M DnaG, 160 nM DnaN2, 20 nM Pol III, 80 nM DnaB6C6, 400 nM DnaA, 40 nM RNaseH, 200 nM ligase, 200 nM Pol I, 200 nM gyrase (GyrA2B2), 20 nM Topo IV (ParC2E2), 200 nM Topo III and 200 nM RecQ). The reaction mixture (final 10 l, unless otherwise noted) included 5 × RCR buffer (2 l) and 4 × Enzyme mix (2.5 l) and was assembled on ice.

After addition of oriC-containing circular DNA, the reaction was incubated at 30°C for the indicated times. $[-^{32}P]$ dATP was included at 40-100 cpm/pmol of total deoxynucleotides when indicated. The reaction was stopped by addition of an equal volume of 2 × Stop buffer (50 mM Tris-HCl pH8.0, 50 mM ethylenediaminetetraacetic acid, 0.2% sodium dodecyl sulphate, 0.1 mg/ml proteinase K, 10% glycerol and 0.2% bromophenol blue), and further incubated at 37°C for 30 min followed by phenol-chloroform extraction. An aliquot (2 l) was analyzed by 0.5% agarose-gel electrophoresis followed by SYBR Green I staining (Molecular Probes) or by phosphor imaging. The images were acquired with a Typhoon FLA 9500 (GE Healthcare). The 4 × Enzyme mix can be stored at -80∘C after rapid freezing with liquid nitrogen and its activity is retained even after five freeze-thaw cycles.

Specifically, the transposition reaction was performed using 100ng of purified DNA by incubating the reaction mix at 55°C for 7 min. Subsequently, 1µl of transposed product mixture was amplified using the Amplification Reaction specified in the OriCiro Cell-Free Cloning System Kit (OriCiro Genomics). The reaction mixture was then incubated at 33°C for a period of 6-16 hr. The amplified products (eccDNA or ecDNA) were analyzed by 0.5% agarose gel electrophoresis and staining protocols known to one of ordinary skill in the art, (e.g. ethidium bromide staining or staining with SYBR safe DNA gel stain obtained from Thermo Fisher). Images were acquired using Chemidoc (Biorad).

In certain embodiments the extracted cellular DNA is further treated with PacI and ExoV, wherein PacI cleaves circular mitochondrial DNA and Exo V further digest linear DNA products. For PacI treatment, 1ug of DNA was treated with 15U PacI (NEB) for 1hr at 37°C. For EvoV treatment, 1ug of DNA was treated with 10U for 30min at 37°C. In samples wherein both enzymes were applied, PacI treatment was performed followed by ExoV treatment. Results of these experiments are shown in FIG. 11, wherein treatment with PacI and Exo V provide for higher resolution of amplified products.

The circular DNA products were further purified from amplified products via phenol/ chloroform extraction. For sequencing, multiple protocols can be used. For example, Illumina Novaseq 6000 (Illumina sequencing) can be used to sequence the amplified and purified products. For Illumina sequencing, 10-100ng of DNA were sheared to 400bp using a LE220 focused-ultrasonicator (Covaris) and then subject to size selection using a SPRI bead system (Beckman Coulter). The fragments were further treated with end-repair, A-tailing, and ligation of Illumina unique adapters (Illumina) using the KAPA Hyper Prep Kit (Illumina) (Roche). The now linear isolated products were further subjected to 5-10 cycles of PCR amplification. Libraries were then prepared by sequencing using 2x150bp on an Illumina Novaseq 6000 sequencing system.

For sequencing, long read sequencing can also be used to characterize the amplified products. For example, the circular DNA products were further purified from amplified products via a phenol/ chloroform extraction. 1µg-5µg DNA samples were digested using the enzymes Srfl (NEB) or PmeI (NEB) to linearize the circular DNA prepared from transposition cassettes that were based on SEQ ID:3 through SEQ ID:6 DNA oligo inserts. The digested DNA was first treated with NEBNext FFPE Repair Mix and then ligated with an adaptor using Ligation Sequencing Kit SQK-LSK110 (Oxford Nanopore Technologies). The linearized DNA were then ligated to an adaptor using a Ligation Sequencing Kit SQK-LSK110 (Oxford Nanopore Technologies). The libraries were then sequenced on the flowcell R9.4.1 (FLO-MIN106, ONT) on a GridION (ONT) using the MinKNOW software for 48 hr.

Alternatively, for long read sequencing, a guide RNA was designed to target the OriC sequences to linearize the circular DNA instead of treatment with restriction enzymes. For this approach, the purified DNA was first dephosphorylated using Quick Calf Intestinal Phosphatase (NEB). After dephosphorylation, the Cas9 complex was introduced where the target OriC site was to be cut. The linearized DNA will be ligated the adaptor using Ligation Sequencing Kit SQK-LSK110 (Oxford Nanopore Technologies). The libraries can be sequenced on the flowcell R9.4.1 (FLO-MIN106, ONT) on a GridION (ONT) using the MinKNOW software for 48 hr.

For sequencing performed using Sequel II from Pacific Biosciences, DNA was prepared as follows. A library of these sequences can be constructed using PacBio SMRTbell Express Template Prep Kit 2.0 and the SMRTbell Enzyme Cleanup Kit (Pacific Biosciences, Menlo Park, CA). Briefly, 1 µg of Srf1-digested DNA is treated for DNA damage repair and end repair/ A-tailing. The repaired/ modified DNA is ligated with SMRTell adapters and purified by Ampure PB using the manufacturer's instruction. The resulting adapter ligated library is subsequently treated with Exonuclease V to remove damaged or non-intact SMRTbell templates. The purified library is then quantified and thereafter sequenced using Sequel II (Pacific Biosciences).

The presence of a nucleic acid the sequence of which was determined according to the methods set forth herein in a sample from a cell or cellular nucleic acid extract was determined as follows. An eccDNA molecule from a cell or cellular nucleic acid extract was converted according to the methods set forth herein to contain an oriC sequence, then amplified and linearized to produce a fragment having portions of the oriC sequence at each end. Following sequence determination from that linearized fragment a pair of primers was produced that, if subject to PCR would produce non-overlapping fragments extended away from one another. Accordingly, any linear fragment subjected to PCR using these primers were not amplified. When these primers were then used for PCR amplification of the eccDNA molecule in the sample from a cell or cellular nucleic acid extract, production of a PCR-amplified product indicated that the sequence was present as an eccDNA in the cell or cellular nucleic acid extract, as illustrated in FIG. 12. PCR was performed using Kapa HiFi HotStart ReadyMix PCR Kit (Roche) under the following conditions: incubation at 98°C for 45sec, followed by 20-30 cycles of 98°C for 15sec, 60°C for 30sec, 72°C for 30sec and a final incubation at 72°C for 1 min. Amplified product is purified using SPRI beads (Beckman) and run on Tapestastion (Agilent) for size confirmation.

Efficient sequencing of eccDNA using the methods of the invention was illustrated as shown. FIG. 13-16 The workflow of this application of the method is shown in FIG. 13. OriC fragments were generated by amplification with Oreo7 (SEQ ID NO. 3) and Oreo8 (SEQ ID NO. 4) primers and digested with restriction enzyme Srfl. Cellular DNA comprising eccDNA, linear genomic DNA and mitochondrial DNA were treated with PacI and Exo V that preferentially digested mitochondrial and linear genome DNA, respectively. OriC was integrated into eccDNA (and so a lesser extent remaining linear genomic DNA by the action of transposase. OriC-containing eccDNA species were amplified as set forth above, linearized, and subject to nucleotide sequencing protocols (e.g., NGS). A library of these sequences was constructed as follows. 1μg samples were digested using the enzymes SrfI (NEB) to linearize the amplified circular DNA. The digested DNA was then first treated with NEBNext FFPE Repair Mix and then ligated with an adaptor using Ligation Sequencing Kit SQK-LSK110 (Oxford Nanopore Technologies). The linearized DNA was thereafter ligated to an adaptor using a Ligation Sequencing Kit SQK-LSK110 (Oxford Nanopore Technologies). The libraries were then sequenced on the flowcell R9.4.1 (FLO-MIN106, ONT) on a GridION (ONT) using the MinKNOW software for 48 hr.

FIG. 14 shows the outcome of these protocols prior to nucleotide sequencing, resulting in three different preparations having a molecular size of about 2kb. After sequencing statistics were analyzed as shown in FIG. 15. The right-hand graph represents sequencing statistics for all species whereas the left-hand graph shows the sequencing statistics for a specific aligned human sequence, hg38. The specific sequence had a higher median read length (1,864bp) than was obtained for all species (1027bp). In addition the read lengths for hg38 were less heterogeneous. Alignment of read sequences to the human reference genome CHM13 T2T v1.1 is shown in FIG. 16 are of eccDNA origin based on their alignment coordinates. All reads generate are also clustered and de novo assembled to determine their consensus sequences and structural features (consensus sequences obtained using these methods are set forth as SEQ ID NOs: 8, 9 and 10).

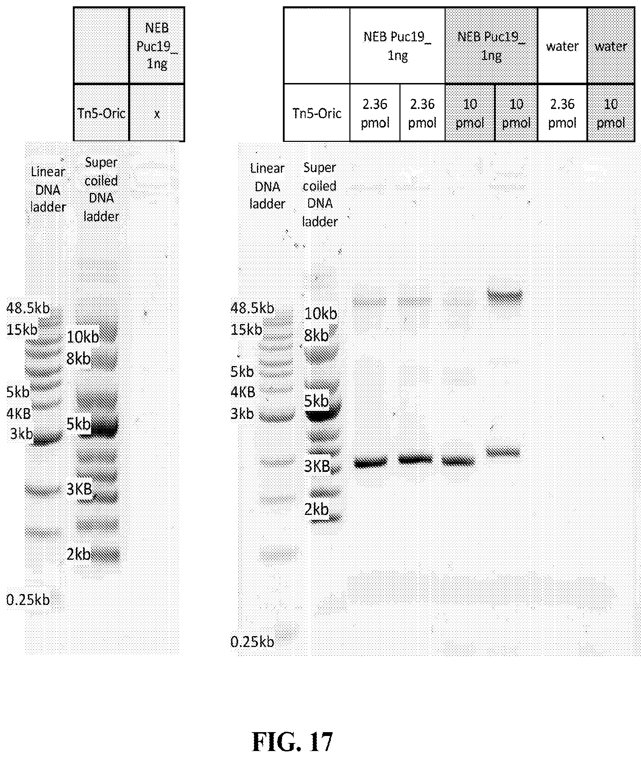

To further establish that the OriC based transposase methods disclosed herein would amplify circular DNA, plasmid pUC19 was used in the presence (righthand side of FIG. 17, showing treatment of pUC19 with either 10pmol or 2.36 pmol transposase) and absence (lefthand side of FIG. 17) of Tn5-OriC. Tn5-OriC treated plasmids were amplified while plasmids untreated with Tn5-OriC were not.

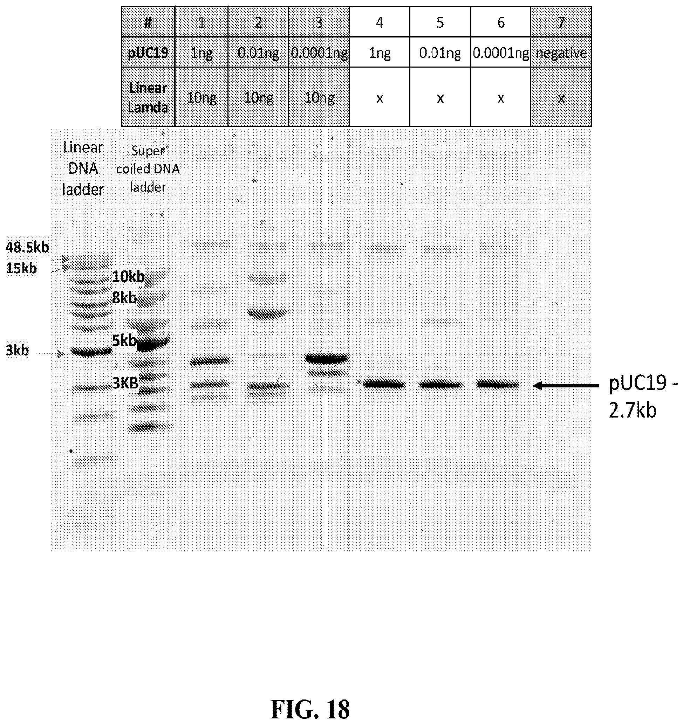

The effect of OriC-mediated amplification on circular and linear DNA was further illustrated in FIG. 18, wherein Tn5-OriC treated circular pUC19 was amplified in the presence (lanes 1-3) and absence (lanes 4-6) of lambda phage DNA fragmented by PmeI treatment. Circular pUC19 DNA was efficiently and specifically amplified, while lambda phage DNA produced various, non-specific fragments. These results also showed that more aggressive EvoV nuclease treatment may be necessary to reduce amplification of linear DNA.

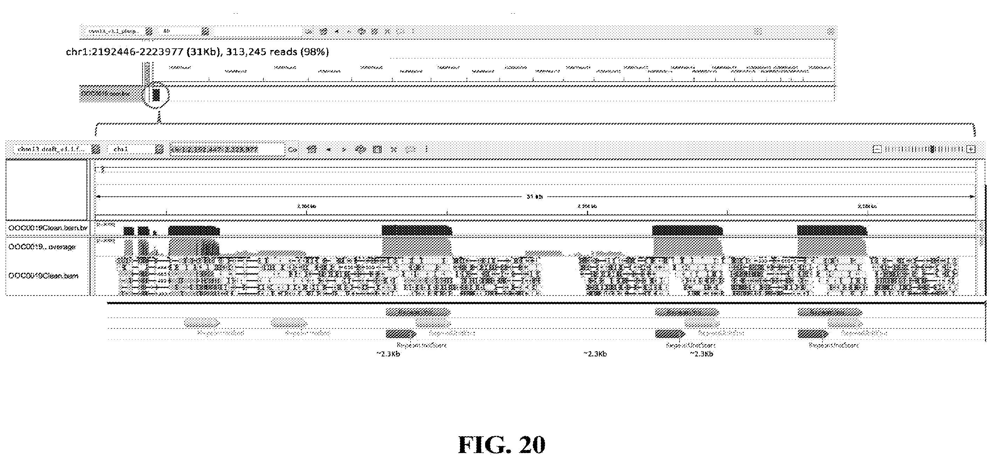

Amplification of circular DNA isolated from cells transfected with a 5.5kb plasmid is shown in FIG. 19. DNA from PC3 cells (a human prostate cancer cell line, available inter alia from ThermoFisher Scientific) without transfected plasmid amplified predominantly mitochondrial DNA, while cells comprising plasmid amplified (predominantly) circular plasmid DNA. At the highest tested concentrations of DNA from cells not transfected with plasmid DNA a 2.5 kb band was detected which arose from human chromosome 1-specific repetitive DNA (shown in FIG. 20). Cellular DNA in these experiments was not treated with PacI, which linearizes mitochondrial DNA, explaining the predominance of amplified mitochondrial DNA in untransfected PC3 genomic DNA samples. The 2.5 kb circular DNA detected in amplification of untransfected PC3 genomic DNA was sequenced and the sequence aligned with human DNA from the T2T (telomere-to-telomere) genomic DNA sequence. The alignment, shown in FIG. 20, revealed that these sequences were from chromosome 1-specific repeats, consistent with earlier reports that repetitive DNA could be detected in eccDNA.

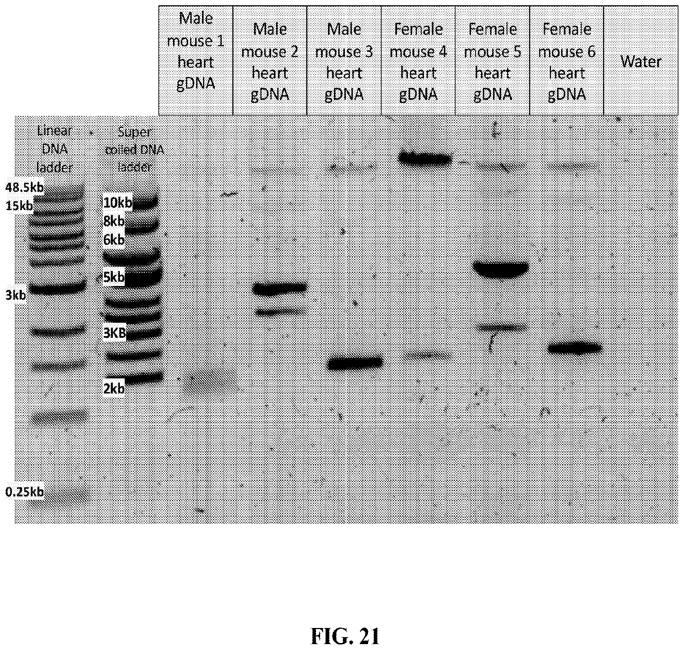

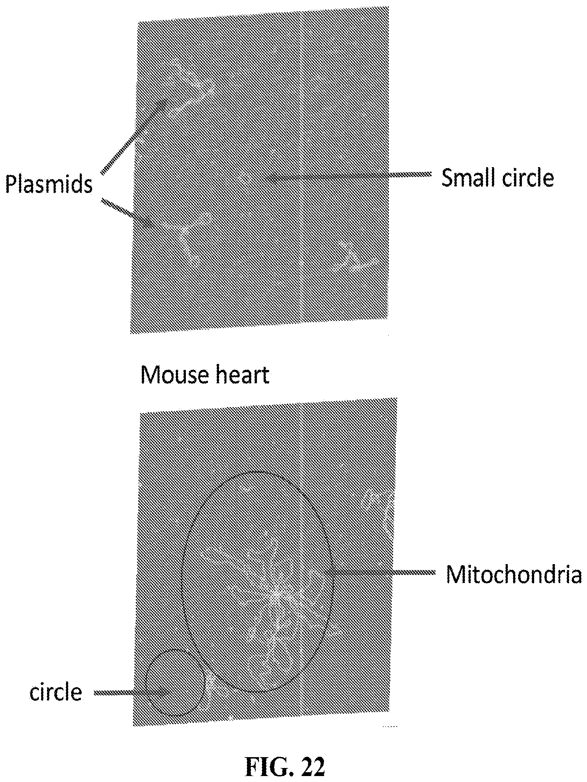

To evaluate whether circular DNA could be detected from normal animal tissue, DNA was extracted from heart muscle from 6 C57Bl mice (3 males and 3 females) and subjected to OriC-mediated amplification as disclosed herein. The results, shown in FIG. 21, showed heterogeneous production of amplified circular DNA species from these individuals, illustrating the presence of circular DNA (eccDNA) species in animal cells in vivo. To establish that the circular DNA species detected in mouse heart as shown in FIG. 21 were in fact endogenous eccDNA species, atomic force microscope images from plasmid-transfected PC3 cells as shown in FIG. 19 and DNA from mouse heart as shown in FIG. 21 are shown in FIG. 22.

Amplification conditions were interrogated under circumstances where a plurality (5) plasmids were amplified together in a single reaction. The plasmids used in this experiment are shown in the table in FIG. s 23A and 23B, and amplification using the methods disclosed herein in 5 different experiments comprising the 5 different plasmids are shown in the lefthand panel at 33°C. The same experiments were performed at 33°C, 36. °C, or 40°C are shown in the righthand panel. This FIG. illustrates that the plasmids were inconsistently amplified at 33°C, but that these plasmids were more evenly and consistently at 36.5°C.

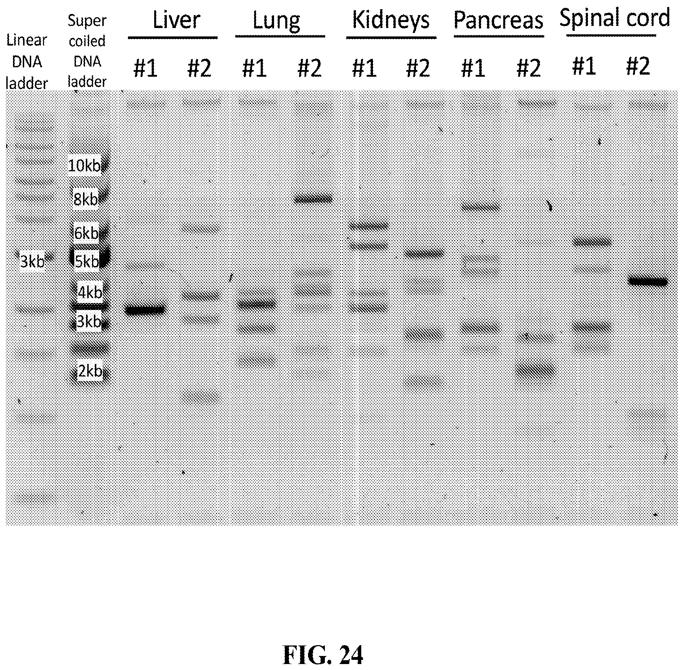

Various tissues from two separate mice were analyzed for the presence of eccDNA using the methods set forth herein. As shown in FIG. 24, various species of eccDNA were detected in each tissue in each mouse, wherein the detected species differed between the different tissue in the same mouse and the tissues of the different mice.

FIGs. 25A through 25F is a summary illustrating the invention disclosed herein. FIG. 25A shows transposase-mediated insertion of OriC into pBeloBAC 11, followed by RCR amplification and detection of the amplified circular DNA. FIG. 25B illustrates a similar experiment using pUC19 and pAT plasmids producing analogous results, showing that circular DNA species of different sizes can be amplifies using the methods set forth herein. FIG. 25C shows the locations in pUC19 plasmids of OriC insertion using decreasing amounts of Tn5-OriC that resulted in fewer and more specific insertion sites for the origin. FIGs. 25D illustrates in a diagram and FIG. 25E the experimental results shown in FIG. 19. FIG. 25F illustrates the presence of circular DNA species detected by atomic force microscopy, and FIG. 25G is a photomicrograph of histochemical staining of the 2.5Kb eccDNA species detected in FIG. 19 in PC3 cells by the methods disclosed herein.

Those skilled in the art will recognize or be able to ascertain using no more than routine experimentation, many equivalents to the specific embodiments of the invention described herein. Such equivalents are intended to be encompassed by the following claims.

SEQ ID NO: 8

CTTGTACTTCGTTCAGTTACGTATTGCTGTACTTCGTTCAGTTACGTGTGCTGGGCAGATGTGTATAA
GAGACAGGCTTGGCTGGCTTGGCTGGCTGGGTGGCTTGCCTGGCTTGGCTGGCTTGGCTGGATGGCTG
GCTTGACTGGCTTGGCTCGCTGGCTGCCCTGGCTGGCTGGATGGCTGGCTGGCTGGCTGGCTTGGCTG
GCTGGCTGGCTGGCTGGCTTGGCTGGCTGGCTGGCTTGGCTGGCTTGGCTGGCTGGCTGGCTTGGCTG
GCTGGCTGGCTTGGCTGGCTGGCTGGGCTGGCTGGCTGGCTGGCTTTGGCTGCCCTGGCTTGGCTGGC
TCTGGCTGGCTGACTGGCTGGCTGGATTGGCTAGCTTGGCTGGCCGGCTGGCTTGGCAGGCTGGATGG
CTCTGGCTGGCTTGGCTGGCAGGCTGGTTTCTCTCTTGGGTGTCTTGGCTGGCTTGGCTGGCTTGGCT
GGCTGGCTCTCTGGCTCTGTGGCTGGCTGGCTGGCTGGCTGGCTTGGCTGGCTGGCTTGGCTGGCTTG
GCTGGCTTGGCTGGCTGGCTTGCTTGGCTGGCTGGCTTGGCTGGCTCTGGCTGGCTTGGCTGGCTGGC
TGGCTTGGCTGGCTTGGCTGGCTGGCTGGCTTGGCTGGCTCTGGCTGGCTTGGCTGGCTGGCTGGCTG
GCTGGCTGGCTGGCTGGCTGGCTGGCTGGCTGGCTGCTGGCTGGCTTGGCTGGCTGGCTGGCTGGCTG
GCTTGGCTGGCTGGCTGGCTGGCTGGCTGGCTGGCTGGCTGGCTGGCTGGCTGGCTGGCTGGCTGGCT
GGCTGGCTGGCTGGCTGGCTGGCTGGCTGGCTGGCTGGCTGGCTGGGCTGGCTGGCTGGCTGGCTGGA
GAGCTGGCTGGAGAGAGAGAGCTGGAGAGAGAGAGCTTGGAGAGAGAGAGAGAGCTGGCTGGCTGG
AGAGCTGGAGAGAGAGAGAGAGAGAGAGAGGCTGGCTTGAGAGAGAGAGAGAGAGAGAGAGAGAGA
GAGAGAGAGAGAGAGAGAGAGAGAGCTGGAGAGAGAGAGAGAGAGAGAGCTGGCTTGGCTGGCTGG
CTGGCTGGAGAGAGAGAGAGAGAGCTGGCTTGGCTGGCTGGCTGGAGAGAGAGAGAGAGAGAGAGAGA
GAGAGAGAGCTGGCTGGCTGGAGAGAGAGCTGGCTGGAGAGAGAGAGAGAGAGAGAGAGAGAGAGAGA
GAGAGAGAGAGAGAGAGAGAGAGAGAGAGAGAGAGAGAGAGAGAGAGAGAGAGAGAGAGAGAGAGAGA
GCTTGGAGAGAGGAGAGAGAGAGGCTGGCTGGAGAGAGAGAGAGAGAGAGAGAGAGAGAGAGGCTGAGAG
AGAGAGCTGGAGAGAGAGAGAGAGAGCAGAGAGAGAGAGAGAGAGGAGAGAGAGAGAGAGAG
AGCTTGGAGAGAGAGAGGAGAGAGAGAGAGAGAGAGAGAGAGAGAGAGAGAGAGAGAGAGAGAGAG
AGAGAGAGAGAGAGAGAGAGAGAGAGAGAGAGCTGGAGAGAGAGAGAGAGAGAGAGGAGAGAGA
GAGAGAGAGAGAGAGAGCTCTGGAGAGAGAGAGAGAGAGAGAGCTGAGAGAGAGGAGGCTGGAGAGAG
AGAGAGAGAGAGAGAGAGAGAGAGAGAGAGAGAGAGAGAGAGAGAGAGAGAGAGAGAGAGAGAGAGAG
AGAGAGACTGAGAGAGAGAGAGAGAGAGAGAGAGAGAGAGAGAGAGAGAGAGAGAGAGAGAGAGAGAG
AGAGAGAGAGAGAGAGAGAGAGAGAGAGAGCTGAGCTGGAGAGAGAGAGAGAGAGAGAGAGAGAGA
GAGAGAGAGAGAGAGAGAGAGAGAGAGAGAGAGCTGAGAGAGAGAGAGAGAGAGAGAGAGAGAGAG
AGAGAGAGAGAGAGAGAGAGAGAGAGAGAGAGAGAGAGAGAGAGAGAGAGGCTCTCTCTGAGAG
AGGCTGGAGAGAGAGAGAGCTTGAGAGAGCTGGCTGGCTGGAGAGAGAGAGAGAGAGAGAGAGAGAGA
GAGAGAGAGAGAGCTGGAGAGAGAGAGAGAGAGAGAGAGAGAGAGAGCTGGAGAGAGCTC
TGCTCTCTCTGGCTTGGCTGGCTGGCTGGCTGAGAGAGAGCTGGCTGGCTTGGCTGGCTGGCTG
GCTGGCTCTGAGAGAGCTGAGCTGGAGAGAGAGAGCTCTGGCTGGCTGGCTTGGCTGGCTCTGGCT
GGCTGGCTGGCTGGCTGAGCTGGCTGGCTGGCTGGAGAGAGAGAGCTGGCTCTGGCTGGAGAGAGACT
GGCTGGCTGGCTGGCTGAGCTGGCTCTGGCTGGCTGGCTGGCTGGCTCTGGCTGGCTCTCTGAGAGAG
AGAGAGCTGGCTGGCTGGAGAGCTGAGCTCTCTGGCTGGCTGGCTGGCTGCTGGCTGGCTGCTGGCTG
AGAGAGCTGAGCTGGCTGGCTGGCTCTGGCTGAGGCTGAGAGGCTTGGCTGGCTGGCTGGAGAGAGGA
GCTGGCTGGCTGGCTGGCTGGGCTTGGCTGGCTGGCTGGCTGGCTGGCTGGCTGAGCTGGAGCTGAGA
GAGAGGCTGAGCTGAGGCTGCTGGCTGGCTTGGCTGGCTGCTCTGCTGCTGGAGCTGGCTGGCTGGCT
GGCTGGCTGGCTCTGGCTGGCTGGCTGGCTCTGCTGGCTCTGGCTGCTGGCTGGCTGGCTGGCTGGCT
GGCTGGCTGGCTGGCTCTGGCTGGCTGGCTTGGCTGGCTGGCTATGCTTGGCTGGCTGGCTGGCTGGC
TGGCTTGGCTGAGAGCTGGCTGGCTGGCTGGCTGGCTGGCTGGCTGGCTCTGCTGGCTGGCTCTGCTG
GCTGAGCTGGCTTGGCTGAGCTCTCTGCTGGCTGGCTGGAGCTTGGCTGGAGCTGGCTTGGCTGAGAG
CTGGCTGGCTGGCTGGCTGGCTTGGCTGGCTGGCTGGCTTGGCTGGCTGGCTGGCTGGCTGGCT
GGCTGGCTGGCTGGCTGGCTTGGCTGGCTGGCTGGCTGGCTGGCTTGGCTGGCTGGCTGGCTGGCTGA
GCTGGCTGGCTGGCTCTGGCTGGAGCTGGCTGGCTGGCTGGCTGGCTGGCTGGCTGGCTGGCTGGCTG
GCTGATCTTGGCTGGGCTGGCTGGCTGGCTGGCTGGCTGGCTGGCTGCTGAGCTTGGCTGGCTGGCTT
GGCTGGCTGCTGGCTGGCTTGGCTGGCTGGCTGAGCTGGATGGCTGGCTGGCTGAGCTGGCTGGCTTG

GCTGGCTGGCTGGCTGGCTGGCTTGGCTGGCTGGCTGCTGGAGCTGGCTGGCTGGCTGGCTGGCTGGC
TGGCTGGCTGGCTGGCTGGCTGGCTGGCTGGCTGGCTTGGCTGGCTGGCTGGCTGGCTGGCTGGAGAGAGAG
AGAGCTGGCTGGCTGGCTGGCTTGGCTGGCTGGCTGGCTGGCTGGCTGGCTGGCTGGCTGGAGGCTGG
CTGGAGAGGCTTGGCTGGCTGGCTGGCTGGCTGGCTGGCTGGCTGAGCTTGGCTGGCTTGGCTGGAGA
GAGAGAGAGGCTGGCTGGCTGAGAGCTGGCTGGCTGGCTTGGCTGGCTGGCTGGCTGGCTGGCTGG
AGAGCTGGAGCTGGCTGGCTGGCTGGCTGGCTGGCTGAGAGCTTGGCTGGCTGGCTGGCTGGCTGGCT
GGCTGGAGAGAGAGAGAGAGCTGGCTGGAGAGCTGGCTGGCTGGAGCTGGCTTGGCTGGCTTGGCTGG
CTGGAGAGAGACTGGCTGGCTGGAGAGCTGAGAGCTGGCTGGAGAGAGAGAGAGGCTGGCTGGCTGGC
TGGCTGGCAGCTGGCTTGGCTGGCTGGCTGGCTGGCTGGCTGGCTGGCTGGCTGGCTTGGCTGGCTTG
GCTGGAGAGGCTGGCTGGCTGGCTGGCTGGCTGGAGAGCTGGCTGAGCTGGCTGGCTGGCTGGCTGGC
TGGCTGGCTGGCTGGCTGAGCTGGCTTGGCTGGCTGGCTGGCTGGCTGGCTTGGCTGGCTGGCTGGCT
GGCTGGCTGGCTGGCTGGCTGGCTGGGAGAGAGAGAGAGAGAGAGAGAGAGAGAGAGAGCTGGCTG
GCTGGCTGAGAGAGAGAGGAGCTGGAGGCTGGCTGGCTGGCTGAGCTGGCTGGCTTGGAGCTGGCTGG
CTGGAGAGCTGGCTGGCACTGGCTGGAGAGAGAGAGAGGCTGGCTGGCTGGGAGAGAGAGAGAGAG
AGAGAGAGAGCTGACACACTGGCTGGCTGGCTTGGCTGGCTTGGCTGGCTGTGTGGCTGGCTGGCT
GGAGAGAGAGAGAGAGCTGGAGAGCTGGCTGGCTGAGAGGCTGGCATGAGCTGGCTGAGCTGGCAGAG
AGGTGGCTGGCTTGGCTGGCTTGAGAGAGAGAGAGAGTGGCTGGCCTGACTGGCTGAGAGAGAGAGAG
CTGTCTCTTATACACATCTGCCCAGCAAGCAATACTTGCAAT

SEQ ID NO: 9

ACTTGTACTTCTTGTACTTCGTTCAGTTACGTATTGCTGGGCAGATGTGTATAAGAGACAGGCTTGGC
TGGCTTGGCTGGCTGGGTGGCTTGCCTGGCTTGGCTGGCTTGGCTGGATGGCTGGCTTGACTGGCTTG
GCTCGCTGGCTGCTGGCTGCTGGCTTGGCTGGCTGGCTGGCTGCTGGCTTGGCTGGCTGGCTGGCTTG
GCTGGCTGGCTGGCTGGCTTGGCTGGCTGGCTGGCTTGGCTGGCTGGCTGGCTGGCTGGCTTGGCTGG
CTGGCTGGCTGGCTGGCTGGCTTGGCTGGCTGGCTGGCTGGCTTGGATGGCTGGCTGGCTTGGC
TGGCTTGGCTGGCTTGGCTGGCTTGGTGGCTGCCAGCTTGGCAGGCCTTGGCTTGGCTTGCTGCCAGC
TTGCTGGCTTGGCTGGCTGGAGCTGGCTGGCTGGCTTGGGCTGGCTGGCTTGGCTGGCTGGCTGGCTG
GCTGGCTTGGCTGGCAGGCTGGCTTGGCTGGCTCTTGGGTGGGCTGGCTGGGCTGGCTTGGCTGGCTG
GCTGGCTGGCTGGCTGGCTGTGGGGCTGGCTGGCTTGGCTGGCTGGCTGGCTGGCTTGGCTGGGCTGG
CTGGCTGGCTTGGCTGGCTGGCTTGGCTGGCTTGGCTGGCTGGCTGGCTGGTGGCTGGCTGGCTTGGC
TGGCTGGCTGGGCTGGCTGGCTTGGCTGGCTGGCTGGCTGGCTTGGCTGGCTGGCTGGCTTGGCTGGC
TGGCTGGCTGGCTGGCTGGCTGGCTGGCTGGAGCTGGCTGGCTGGCTGGCTGGCTTGGCTGGGGCTGG
CTGGCTGGCTGGCTGGCTGGCTGGCTGGCTGGCTGGCTTGGCTGGCTTGGCTGGCTGGCTGGGC
TGGCTGGGCTGGCTGGCTGGCTGGCTGGCTGGCTGCCAGCTGGCTGGCTGGCTGGCTGGCTGGCTGGC
TGGCTGGCTGGCTGGGCTGCTGGCTTGGCTGGCTGGCTGGCTGGCTGGCTGGCTGGCTTGGCTGGCCA
TGGCTGGCTGGCTGGCTGGGCTGGCTGGCTTGGCTGGCTTGGCTGGCTGGCTGGCTGGCTGGCTGGCT
GGCTGGCTGGCCAAGCTGGCTGGCTGGCTGGGCTGGCTGGCTGGCTGGGCTGCAGCTGGCTGGCTGGC
TGGCTGGCTGGAGAGAGCTGCAGCTGGCTGGCTGGCAGCCTGCTTGGCTGGCTGGCTGGCTTGGCCAG
CCAGGCTGGCTGGAGAAGCCAGCTTGGCTGGCTGGCCAGCTGGGATGGCTAGCTGGCTTGGAGCCAGC
TGGAGAGGAGCCTGGCTGGCTGGCTGGCTTGGCTGGCTGGCTGCTTGGCTGGCTGGCTGGCTGGAGCC
TGGCTGGCTGCTTGAGCTGGCTGGCTGGCTGGCTGGCTGGCTGGCTGGCTGGCTGGCTGAGCTGGCAG
CTGGCTGGCTGGCTGGCTGGCCAGCAGCAGCCAGGCAGCTGGCTGGCTGGCTGGCTGGCAGCTTGGCT
GGCCAGCTTGGCTGAGAGAGCTGGCTGGCTGGCTGGCAGCCAGCCTGGCTTGGCTGGCTGGCTGGCCA
GGCTGCTGGCTGGCAGCTGGCTGGCTTGGCTGGCTGGGCTGGCTGGCTTGGCTGGCTTGGCTGGCTGG
CTGGCTGGCTGGCTGGCAAGGCTGGCAGCCTGGCTGGCCAGCTGGCTGGCTTGGCTGGCCTGGCAGCT
GGCTGGCCAGCTGGCTGGCAGCCAGCTGGCCCAGCCTGGCTGGCTGGCAGCCAGCCAGCTGGCCAGCT
GCTGGCTGGCAGGCTGCTGCTGGCTGGCTGGCTGGCCTGGCTGGCTGGCCTGGCTTGGCCTTGGCTGG
CTGGCTGGCTGGCTGGCTGGCTGCTGCAAGCCAGCTGGCCTGGCTGCCTGGCCTGGCTTGGCTG
GCTGGCTGGCTTGGCTGGCTGGCTGGCTGGCTGGCTGGCTTCTGGCCAGGCTGGCTGGCTTGGCTGGC
TGGCCTGGCCAGCCAGCTGCTGGCAAGGCTGGCTGGCAGCCAGCTGGCTGGCCAGGCTGGCCAGCAGC

```
TGGCTGGCTGGCTGGCTGGCTGGCTGAGCCTGGCTGGCTGGCTGGCTGGCCAGCTGGCTGCCAAGCCT
GGCTGGAGGCCTGGCTGGCTTGGCTGGCTTGGCTTGGCTGGCTGGCCAGCCAGGCCAGCCAGCTGGCT
GGCCTGGAGCCTGCCTGGAGTTAATGAGCTGGCTTGGCTGCCAGCTGGCCTGGCTGGCTTGGCTGGCT
TGGCTGGCTGGCTGGCTGGCTGGCTGAGGCTGGCTGGCTGGCTGCCAGCTGGCTGGCTGGCTGGCCAG
GCTGCAGCTGAGCTGGCAGGCTTCTGGCTGGCTGCTTGGCTAGCTGGCTGGCTGTCTCTGGCTCTGGC
TGGCTGGCTGGCTGGCCAGCCTGGCAGCCTGGCAGCCAGCTGGCTTGGCTGGCTGGCTCTGGCTGGCT
GGCTGGCTGCCAGCTGGCTGGCTTCAGCTGGCTGGAGCAGATCTCTCTCCAGCCAGCCAGCAGCAGAG
CTGGCCAAGCCAGCTGGCTGGCAGAGCCAGCCAGCAAGCATCAGCCAGCCAGAGCCAGAGAGCAGAGC
CTGCTGCCTGAGAGAGAGAGAGCCAGAGATCAGAGAGCTGAGAGAGAGAGAGAGAGAGAGAGAGAGAG
AGAGCTCAGCCAGAGAGAAAGCCAGCTGCCAGCCACAGAGAGAGAGCTGCCAGAGAGAGAGAGCCAGA
GAGAGAGAGAGAGAGAGAAAGAGAGAGAGCTCTGGCTGGCTTGGCAGAGAGAGCCAGAGGCCAGCCAG
AGCTGGCTCTGCCAGCCAGAGAGAGAGAGAGCTCCAGAGAGCTGGCTGAGCAGAGCAGCTGGAGAGAG
AGAGAGAGCAGCCAGGCTGAGCAGCTGGCAGGCTGGCAGCCTGCCAGCTCTGAGCAGAGCTCAGAGCT
GAGCTCTCTCTCTGGCTGAGAGAGCTGGAGAGAGAGAGAGAGAGAGACCAGCCAGAGAGAGCCAGAGC
CAGCCAGAGAGCTGGCACAGCAGAGAGAGCTTGAGAGACAGAGAGAGAGAGAGACAGAGAGAGAGAGA
GAGAGCCAGAGAGAGAGAGAGAGCCAGACAGAGAGAGAGAGAGAGAGAGAGAGAGAGCCTGAGAGA
GAGAGAGAGAGGCTGAGAGAGCCAGAGAGAGAGAGAGAGCTGAGAGAGAGAGAGAGAGCCTCTCTC
TCTGCCAGCTGAGAGAGAGGCTCTCAGAGCCAGCTGGCTCAGCAGAGAGACTCTCTGAGAGAGA
CAGAGAGCTGAGCTCTGAGCTGGCTGAGAGAGAGAGAGAGAGAGAGAGAGAGAGAGAGAGAGAGAG
CTCTCTCAGAGAGAGAGAGAGAGAGAGAGAGAGCTCTCTGAGCTTGAGAGAGAGAGAGAGAGAGCCAGAG
AGACAGCCAGCTGGAGAGAGAGAGAGAGAGAGACAGGACAGCACACACTGAGCTCCCAGAGAGAGAGAGA
GAGAGAGAGAGAGAGAGAGAGAGAGAGAGAGAGAGAGAGAGTGAGAGAGAGAGAGAGAGCTGAGAGAGAG
AGAGCAGCTTGGCTGAGCTGAGAGAGAGAGCTGAGAGAGAGAGAGCCTGAGAGCACAGAGGCTGGCTG
AGAGAGAGAGAGAGAGAGAGAGAGAGAGAGAGAGCTTGGAGAGAGAGAGAGAGAGAGAGAGAGAGAGA
CAGAGCAGCCAAGAGCAGCTGGCTCTGAGAGAGCTGAGCTGAGCTGGCTCTGGCTGGCTGCTGGCTGG
AGAGAGAGAGAGAGAGAGAGAGAGAGAGAGAGAGCCAGAGAGAGAGAGAGAGCAGAGAGAGAGAGAGC
TGACAGAGAGAGAGACAGAGAGCCTTGGCTGAGAGAGAGAGAGAGAGAGAGAGAGAGAGAGAGAGAGAGA
AGAGAGAGAGAGAGAGAGAGAGAGAGCTGTCTCTTATCTTATACACATACTGCCCAGCAATCTGCC
CAGCAATACGTAAGAGA
```

SEQ ID NO: 10

```
ACAAACATACTTGTACTTCGTTCAGTTACGTATTGCTGGGCGTGTACTTCGTTCAGTTACGTATTGCT
GGGCAGATGTGTATAAGAGACAGCCATCTAGCCATCCAGCCAGTCAGGCCAGCCACCCAGCCAAGCCA
GCCAAGCCAGCCAAGCCAGCCACCCGCCAAGCCAGCCAGCCAGCCATGCCAGCCAGCCAGCCAGCCAG
CCAGTTACAAGCCCAGTATTGCCTGCTTCGTTCAGTTACGTATTGCTGGGCAGATGTGTATAAGAGAC
AGCCATCTAGCCATCCAGCCAGTCAGGCCAGCCACCCAGCCAAGCCAGCCAAGCCAGCCAAGCCAGCC
ACCCAGCCAAGCCAGCCAGCCAGCCAGCCAGCCAAGCCAGCCAGCCAGCCAGCCAAGCCACCCAGCCA
GCCAGCCAAGCCACACAGCCAGCCAGCCAGCCAAGCCAGCCAGCCAGCCAGCCAGCCAGCCAGC
CAGCCAGCCAGCCAGCCAGCCAGCCAGCCCAGCCAGCCAGCCAGCCAGCCAGCCAGCCAGCCAG
CCAGCCAGCCAGCCAGCCAGCCAGCCAGCCAGCCAGCCAGCCAGCCAGCCAGCCAGCCAGCCAG
CCAGCCAGCCCAGCCAGCCAGCCAGCCAGCCAGCCAGCCAGCCAGCCAGCCAGCCAGCCAGCCA
GCCAGCCAGCCAGCCAGCCAGCCAGCCAGCCAAGCCAGCCAGCCAGCCAGCCAGCCAGCC
AGCCAGCCAGCCAGCCCAGCCAGCCAGCCAGCCAGCCAGCCAGCCAGCCAGCCAGCCAAG
CCAGCCAAGCCAGCCAGCCAGCCAAGCCAGCCAGCCAGCCAGCCATCCGAGCCAG
CCAGCCAGCCAGCCAAGCCCAGCCAAGCCCAGCCAGCCAGCCATCCCAGCCAGTCAAGCCAGCCAAGC
CAGCCAAGCCAGCCAGCCAGCCAAGCCAGCCAGCCAGCCAGCCAGCCAGCCAGCCAAGCCAGCCAAGC
CAGCCAAGCCAGCCAGCCAGCCAGCCAAGCCAGCCAAGCCAGCCAGCCAGCCAAGCCAGCCAAG
CCAGCCATCCAGCCAGCCAGCCAAGCCAGCCAAGCCAGCCCAGCCAGCCAAGCCAGCCAGCCAAGCCA
GCCAGCCCAGCCAAGCCAGCCAGCCAGCCAGCCAGCCAGCCAGCCAGCCAGCCAGCCAAGCCAGCCAA
GCCAGCCCAGCCAGCCAGCCAGCCAGCCAGCCAGCCAGCCAGCCAGCCAAGCCAGCCAGCCAGCCAGC
```

```
CAGCCAGCCAGCCAGCCAGCCAGCCAAGCCAGCCAGCCAGCCAAGCCAGCCAAGCCAGCCAAGCCAGC
CAGCCAGCCAGCCAGCCAAGCCCCAGCCTCAAGCCAGCCAGCCAGCCAAGCCAGCCAGCCAGCCAGCC
AGCCAGCCAGCCAGCCAAGCCAGCCAAGCCAAGCTCTTATACACAGCCAGCCAGCCAGCCAGCCAGCC
AAGCCAGCCAGCCCAGCCAGCCAGCCAGCCAGCCAGCCAGCCAGCCAACCAGCCAGCCAGCCAGCCAG
CCAAGCCAGCCAGCCAGCCAGCCAGCCAGCCAAGCCAGCCAGCCAGCCATCCAGCCAAGCCAGCCAGC
CAGCCAAGCCAGCCCAGCCAGCCAAGCCAGCCAAGCCTGTCTCTTAGCCATCTGCCCAGCCTAGCCAG
CCCAGCCAGCCAGCCTTTTTCAGCCAAGCCAGCCAAGTACCAATCCCTCATTCTGACCCAGCTGCCAG
CCATCATTTGCCAGCCAGCCAAAACCTGGGCAGCCAAGCCAGCCAAGCCAGCCTAATGATCCTTTTCC
AGGTTGTTGATCAAGCCAGCCAAGCAGCCAAGCCAGCCAGCCAGCGAAAGATCATTAGCCTGTGAAGA
TCAGTGATCCAGATAAGCCTGGGATCAGCCAGCCATACACAAGCCAAAGCCATCCAAGCCAGTTCTTT
GGATAACTACCGGTTGAGCCAAGCTTCCAGTCAAGACAGCCATCCAGTTATCCAGCCAAGCCTTTAGC
ACAACATACTAGCCAGCCAAGCCAGCCAAGCCTGTCTCTTATACACATCTGCCCAGCAATACGTATGC
AATGTGAT
```

## Claims

1. A composition comprising a plurality of eukaryotic extrachromosomal circular DNA (eccDNA) or eukaryotic circular DNA isolated from a cancerous cell (ecDNA), wherein the circular DNA species have been modified by insertion of a prokaryotic origin of replication by a transposase, wherein the transposon is assembled with a set of linear DNA oligonucleotide primers which comprise:

   - SEQ ID: 1 and SEQ ID: 2; or
   - SEQ ID: 3 and SEQ ID: 4; or
   - SEQ ID: 5 and SEQ: ID: 6.

2. The composition of circular DNA of claim 1, wherein:

   i) the modified plurality of circular DNA species is capable of being amplified by replication *in vitro* mediated by said prokaryotic origin of replication; and/or
   ii) the origin of replication is *E. coli* OriC; optionally wherein said plurality of circular DNA results from isothermal replication cycle reaction (RCR) amplification *in vitro;* and/or
   iii) the eukaryotic cell is a human cell.

3. A method of performing next generation DNA sequencing on circular DNA isolated from a eukaryotic cell, wherein said circular DNA comprises the plurality of circular DNA according to any one of claims 1-2.

4. The method of claim 3, wherein:

   a) the eukaryotic circular DNA is linearized with Cas9 or a restriction enzyme;
   and/or
   b) the circular DNA is:

      i) human eccDNA or human cancerous ecDNA; or
      ii) isolated from a biofluid.

5. The method of claim 4, wherein:

   option b-i) applies and the circular DNA is isolated from a tissue, wherein the tissue is optionally a tumor or biopsy thereof; or
   option b-ii) applies and the biofluid is human blood or human cerebral spinal fluid (CSF).

6. A kit comprising nucleic acid encoding a prokaryotic origin of replication, protein components specific for the prokaryotic origin of replication and capable of replicating DNA comprising said prokaryotic origin of replication, and a transposase capable of inserting the prokaryotic origin of replication into human-derived eccDNA by a transposition reaction, wherein the transposon is assembled with a set of linear DNA oligonucleotide primers which comprise:

- SEQ ID: 1 and SEQ ID: 2; or
- SEQ ID: 3 and SEQ ID: 4; or
- SEQ ID: 5 and SEQ: ID: 6.

**7.** The kit of claim 6, further comprising a purified preparation of Cas9 and associated guide mRNA.

**8.** A method of producing the plurality of eccDNA or ecDNA according to claim 1, the method comprising the steps of

a) isolating cellular DNA comprising circular DNA from the cell;
b) modifying the circular DNA comprising eccDNA or ecDNA by inserting the prokaryotic origin of replication into the isolated circular DNA by a transposase;
c) amplifying *in vitro* the modified circular DNA comprising eccDNA or ecDNA; and
d) isolating the amplified eccDNA or ecDNA;

wherein the transposition reaction utilizes a transposon that is assembled with a set of linear DNA oligonucleotide primers which comprise:

- SEQ ID: 1 and SEQ ID: 2; or
- SEQ ID: 3 and SEQ ID: 4; or
- SEQ ID: 5 and SEQ: ID: 6.

**9.** The method of claim 8, wherein:

a) the origin of replication is *E. coli* OriC; or
b) said plurality of circular DNA results from isothermal replication cycle reaction (RCR) amplification *in vitro*.

**10.** A method of producing the plurality of eccDNA or ecDNA according to claim 1, the method comprising the steps of

a) isolating circular DNA comprising eccDNA or ecDNA from the cell;
b) modifying the circular DNA comprising eccDNA or ecDNA by inserting the prokaryotic origin of replication into the isolated circular DNA by a transposase;
c) applying an Exo V endonuclease to remove linearized non-circular DNA;
d) applying PacI to linearize the circularized mitochondrial DNA;
d) amplifying *in vitro* the modified circular DNA comprising eccDNA or ecDNA; and
e) isolating the amplified eccDNA or ecDNA;

wherein the transposition reaction utilizes a transposon that is assembled with a set of linear DNA oligonucleotide primers which comprise:

- SEQ ID: 1 and SEQ ID: 2; or
- SEQ ID: 3 and SEQ ID: 4; or
- SEQ ID: 5 and SEQ: ID: 6.

**11.** The method of any one of claims 8-10, wherein the circular DNA comprising eccDNA or ecDNA is human; optionally wherein

a) the human eccDNA or ecDNA is isolated from a tissue; optionally wherein the tissue is a tumor or biopsy thereof; or
b) the human eccDNA or ecDNA is isolated from a biofluid, optionally wherein the biofluid is blood or cerebral spinal fluid (CSF).

**12.** A method of identifying a nucleic acid sequence of eukaryotic extrachromosomal circular DNA (eccDNA) or (ecDNA), the method comprising:

i) performing nucleic acid sequencing on a composition according to claim 1;
or
ii) performing nucleic acid sequencing on extrachromosomal circular DNA (eccDNA) or (ecDNA) isolated from a eukaryotic cell, wherein the extrachromosomal circular DNA (eccDNA) or (ecDNA) is obtained according to the method of claim 8;

or

iii) performing nucleic acid sequencing on extrachromosomal circular DNA (eccDNA) or (ecDNA) isolated from a eukaryotic cell, wherein the extrachromosomal circular DNA (eccDNA) or (ecDNA) is obtained according to the method of claim 10.

13. The method of claim 12, wherein:

a) the method includes the further step of linearizing the amplified (eccDNA) or (ecDNA) with Cas9 or a restriction enzyme before sequencing; and/or

b) the method of identifying a nucleic acid sequence is nanopore long-read sequencing.

14. A method of identifying a cancer patient having metastatic disease for treatment, the method comprising assessing a composition of claim 1 for metastasis-specific ecDNA.

15. A method of identifying a cancer patient having metastatic disease for treatment, comprising the steps of:

a) isolating circular DNA (ecDNA) from the patient;

b) modifying the ecDNA by inserting a prokaryotic origin of replication into the isolated circular DNA via a transposition reaction;

c) applying an Exo V endonuclease to remove linearized non-circular DNA;

d) applying PacI to linearize the circularized mitochondrial DNA;

d) amplifying *in vitro* the modified circular DNA comprising ecDNA;

e) purifying the amplified ecDNA;

f) linearizing the amplified ecDNA with a Cas9 enzyme or restriction enzyme;

g) characterizing the amplified product of step f) using a nanopore long-read sequencing technique; and

h) using the characterization of step g) to of identify a cancer patient having metastatic disease;

wherein the transposition reaction utilizes a transposon that is assembled with a set of linear DNA oligonucleotide primers which comprise:

• SEQ ID: 1 and SEQ ID: 2; or

• SEQ ID: 3 and SEQ ID: 4; or

• SEQ ID: 5 and SEQ: ID: 6.

**Patentansprüche**

1. Zusammensetzung, umfassend eine Vielzahl von eukaryotischer extrachromosomaler zirkulärer DNA (eccDNA) oder aus einer Krebszelle isolierter eukaryotischer zirkulärer DNA (ecDNA), wobei die zirkulären DNA-Spezies durch Einfügen eines prokaryotischen Replikationsursprungs durch eine Transposase modifiziert wurden, wobei das Transposon mit einem Satz linearer DNA-Oligonukleotid-Primer zusammengesetzt ist, der Folgendes umfasst:

• SEQ ID: 1 und SEQ ID: 2; oder

• SEQ ID: 3 und SEQ ID: 4; oder

• SEQ ID: 5 und SEQ ID: 6.

2. Zusammensetzung zirkulärer DNA nach Anspruch 1, wobei:

i) die modifizierte Vielzahl von zirkulären DNA-Spezies in der Lage ist, durch Replikation *in vitro* amplifiziert zu werden, die durch den prokaryotischen Replikationsursprung vermittelt wird; und/oder

ii) der Replikationsursprung *E.-coli*-OriC ist; optional wobei die Vielzahl von zirkulärer DNA aus einer isothermen Replikationszyklus-Reaktion(RCR)-Amplifikation *in vitro* resultiert; und/oder

iii) die eukaryotische Zelle eine humane Zelle ist.

3. Verfahren zum Durchführen der DNA-Sequenzierung der nächsten Generation an zirkulärer DNA, die aus einer eukaryotischen Zelle isoliert wurde, wobei die zirkuläre DNA die Vielzahl von zirkulärer DNA nach einem der Ansprüche 1-2 umfasst.

4. Verfahren nach Anspruch 3, wobei:

> a) die eukaryotische zirkuläre DNA mit Cas9 oder einem Restriktionsenzym linearisiert wird; und/oder
> b) die zirkuläre DNA Folgendes ist:
>
>> i) humane eccDNA oder humane Krebs-ecDNA; oder
>> ii) aus einem Biofluid isoliert.

5. Verfahren nach Anspruch 4, wobei:

> Option b-i) gilt und die zirkuläre DNA aus einem Gewebe isoliert ist, wobei das Gewebe optional ein Tumor oder eine Biopsie davon ist; oder
> Option b-ii) gilt und das Biofluid humanes Blut oder humane Cerebrospinalflüssigkeit (CSF) ist.

6. Kit, umfassend Nukleinsäure, die für einen prokaryotischen Replikationsursprung codiert, Proteinkomponenten, die für den prokaryotischen Replikationsursprung spezifisch sind und in der Lage sind, DNA zu replizieren, die den prokaryotischen Replikationsursprung umfasst, und eine Transposase, die in der Lage ist, den prokaryotischen Replikationsursprung durch eine Transpositionsreaktion in humane eccDNA einzufügen, wobei das Transposon mit einem Satz linearer DNA-Oligonukleotid-Primer zusammengesetzt ist, der Folgendes umfasst:

> • SEQ ID: 1 und SEQ ID: 2; oder
> • SEQ ID: 3 und SEQ ID: 4; oder
> • SEQ ID: 5 und SEQ ID: 6.

7. Kit nach Anspruch 6, ferner umfassend eine gereinigte Zubereitung von Cas9 und zugehöriger Guide-mRNA.

8. Verfahren zum Herstellen der Vielzahl von eccDNA oder ecDNA nach Anspruch 1, wobei das Verfahren die folgenden Schritte umfasst:

> a) Isolieren von zellulärer DNA, die zirkuläre DNA umfasst, aus der Zelle;
> b) Modifizieren der zirkulären DNA, die eccDNA oder ecDNA umfasst, durch Einfügen des prokaryotischen Replikationsursprungs in die isolierte zirkuläre DNA durch eine Transposase;
> c) Amplifizieren der modifizierten zirkulären DNA, die eccDNA oder ecDNA umfasst, *in vitro;* und
> d) Isolieren der amplifizierten eccDNA oder ecDNA;

wobei die Transpositionsreaktion ein Transposon nutzt, das mit einem Satz linearer DNA-Oligonukleotid-Primer zusammengesetzt ist, der Folgendes umfasst:

> • SEQ ID: 1 und SEQ ID: 2; oder
> • SEQ ID: 3 und SEQ ID: 4; oder
> • SEQ ID: 5 und SEQ ID: 6.

9. Verfahren nach Anspruch 8, wobei:

> a) der Replikationsursprung *E.-coli*-OriC ist; oder
> b) die Vielzahl von zirkulärer DNA aus einer isothermen Replikationszyklus-Reaktion(RCR)-Amplifikation *in vitro* resultiert.

10. Verfahren zum Herstellen der Vielzahl von eccDNA oder ecDNA nach Anspruch 1, wobei das Verfahren die folgenden Schritte umfasst:

> a) Isolieren zirkulärer DNA, die eccDNA oder ecDNA umfasst, aus der Zelle;
> b) Modifizieren der zirkulären DNA, die eccDNA oder ecDNA umfasst, durch Einfügen des prokaryotischen Replikationsursprungs in die isolierte zirkuläre DNA durch eine Transposase;
> c) Anwenden einer Exo-V-Endonuklease zum Entfernen linearisierter, nichtzirkulärer DNA;
> d) Anwenden von PacI zum Linearisieren der zirkularisierten mitochondrialen DNA;
> d) Amplifizieren der modifizierten zirkulären DNA, die eccDNA oder ecDNA umfasst, *in vitro;* und
> e) Isolieren der amplifizierten eccDNA oder ecDNA;

wobei die Transpositionsreaktion ein Transposon nutzt, das mit einem Satz linearer DNA-Oligonukleotid-Primer zusammengesetzt ist, der Folgendes umfasst:

- SEQ ID: 1 und SEQ ID: 2; oder
- SEQ ID: 3 und SEQ ID: 4; oder
- SEQ ID: 5 und SEQ ID: 6.

11. Verfahren nach einem der Ansprüche 8-10, wobei die zirkuläre DNA, die eccDNA oder ecDNA umfasst, human ist; optional wobei

a) die humane eccDNA oder ecDNA aus einem Gewebe isoliert ist; optional wobei das Gewebe ein Tumor oder eine Biopsie davon ist; oder

b) die humane eccDNA oder ecDNA aus einem Biofluid isoliert ist, optional wobei das Biofluid Blut oder Cerebrospinalflüssigkeit (CSF) ist.

12. Verfahren zum Identifizieren einer Nukleinsäuresequenz eukaryotischer extrachromosomaler zirkulärer DNA (eccDNA) oder (ecDNA), wobei das Verfahren Folgendes umfasst:

i) Durchführen einer Nukleinsäuresequenzierung an einer Zusammensetzung nach Anspruch 1; oder

ii) Durchführen einer Nukleinsäuresequenzierung an extrachromosomaler zirkulärer DNA (eccDNA) oder (ecDNA), die aus einer eukaryotischen Zelle isoliert wurde, wobei die extrachromosomale zirkuläre DNA (eccDNA) oder (ecDNA) gemäß dem Verfahren nach Anspruch 8 erhalten wurde; oder

iii) Durchführen einer Nukleinsäuresequenzierung an extrachromosomaler zirkulärer DNA (eccDNA) oder (ecDNA), die aus einer eukaryotischen Zelle isoliert wurde, wobei die extrachromosomale zirkuläre DNA (eccDNA) oder (ecDNA) gemäß dem Verfahren nach Anspruch 10 erhalten wurde.

13. Verfahren nach Anspruch 12, wobei:

a) das Verfahren den weiteren Schritt des Linearisierens der amplifizierten (eccDNA) oder (ecDNA) mit Cas9 oder einem Restriktionsenzym vor dem Sequenzieren beinhaltet; und/oder

b) das Verfahren zum Identifizieren einer Nukleinsäuresequenz die Long-Read-Nanopore-Sequenzierung ist.

14. Verfahren zum Identifizieren eines Krebspatienten mit metastasierter Krankheit zur Behandlung, wobei das Verfahren das Bewerten einer Zusammensetzung nach Anspruch 1 auf metastasenspezifische ecDNA umfasst.

15. Verfahren zum Identifizieren eines Krebspatienten mit metastasierter Krankheit zur Behandlung, umfassend die folgenden Schritte:

a) Isolieren zirkulärer DNA (ecDNA) aus dem Patienten;

b) Modifizieren der ecDNA durch Einfügen eines prokaryotischen Replikationsursprungs in die isolierte zirkuläre DNA über eine Transpositionsreaktion;

c) Anwenden einer Exo-V-Endonuklease zum Entfernen linearisierter, nichtzirkulärer DNA;

d) Anwenden von PacI zum Linearisieren der zirkularisierten mitochondrialen DNA;

d) Amplifizieren der modifizierten zirkulären DNA, die ecDNA umfasst, *in vitro;*

e) Reinigen der amplifizierten ecDNA;

f) Linearisieren der amplifizierten ecDNA mit einem Cas9-Enzym oder Restriktionsenzym;

g) Charakterisieren des amplifizierten Produkts aus Schritt f) unter Verwendung einer Long-Read-Nanopore-Sequenzierungstechnik; und

h) Verwenden der Charakterisierung aus Schritt g) zum Identifizieren eines Krebspatienten mit metastasierter Krankheit;

wobei die Transpositionsreaktion ein Transposon nutzt, das mit einem Satz linearer DNA-Oligonukleotid-Primer zusammengesetzt ist, der Folgendes umfasst:

- SEQ ID: 1 und SEQ ID: 2; oder
- SEQ ID: 3 und SEQ ID: 4; oder

• SEQ ID: 5 und SEQ ID: 6.

**Revendications**

1. Composition comprenant une pluralité d'ADN extrachromosomique circulaire (ADNec) eucaryote ou d'ADN circulaire eucaryote isolé à partir d'une cellule cancéreuse (ADNecc), dans laquelle les espèces d'ADN circulaire ont été modifiées par insertion d'une origine de réplication procaryote par une transposase, dans laquelle le transposon est assemblé avec un ensemble d'amorces oligonucléotidiques d'ADN linéaires qui comprennent :

   • SEQ ID : 1 et SEQ ID : 2 ; ou
   • SEQ ID : 3 et SEQ ID : 4 ; ou
   • SEQ ID : 5 et SEQ ID : 6.

2. Composition d'ADN circulaire selon la revendication 1, dans laquelle :

   i) la pluralité modifiée d'espèces d'ADN circulaire est capable d'être amplifiée par réplication *in vitro à* médiation par ladite origine de réplication procaryote ; et/ou
   ii) l'origine de réplication est OriC *d'E. coli ;* éventuellement dans laquelle ladite pluralité d'ADN circulaire résulte d'une amplification par réaction de cycle de réplication (RCR) isotherme *in vitro ;* et/ou
   iii) la cellule eucaryote est une cellule humaine.

3. Procédé de réalisation d'un séquençage d'ADN de nouvelle génération sur de l'ADN circulaire isolé à partir d'une cellule eucaryote, dans lequel ledit ADN circulaire comprend la pluralité d'ADN circulaire selon l'une quelconque des revendications 1 et 2.

4. Procédé selon la revendication 3, dans lequel :

   a) l'ADN circulaire eucaryote est linéarisé avec Cas9 ou une enzyme de restriction ; et/ou
   b) l'ADN circulaire est :

      i) un ADNec humain ou un ADNecc cancéreux humain ; ou
      ii) isolé à partir d'un biofluide.

5. Procédé selon la revendication 4, dans lequel :

   l'option b-i) s'applique et l'ADN circulaire est isolé à partir d'un tissu, le tissu étant éventuellement une tumeur ou une biopsie de celle-ci ; ou
   l'option b-ii) s'applique et le biofluide est du sang humain ou du liquide céphalo-rachidien (LCR) humain.

6. Kit comprenant un acide nucléique codant pour une origine de réplication procaryote, des composants protéiques spécifiques pour l'origine de réplication procaryote et capables de répliquer de l'ADN comprenant ladite origine de réplication procaryote, et une transposase capable d'insérer l'origine de réplication procaryote dans un ADNec d'origine humaine par une réaction de transposition, dans lequel le transposon est assemblé avec un ensemble d'amorces oligonucléotidiques d'ADN linéaires qui comprennent :

   • SEQ ID : 1 et SEQ ID : 2 ; ou
   • SEQ ID : 3 et SEQ ID : 4 ; ou
   • SEQ ID : 5 et SEQ ID : 6.

7. Kit selon la revendication 6, comprenant en outre une préparation purifiée de Cas9 et d'ARN messager ARNm guide associé.

8. Procédé de production de la pluralité d'ADNec ou d'ADNecc selon la revendication 1, le procédé comprenant les étapes consistant à :

   a) isoler l'ADN cellulaire comprenant de l'ADN circulaire à partir de la cellule ;
   b) modifier l'ADN circulaire comprenant de l'ADNec ou de l'ADNecc en insérant l'origine de réplication procaryote dans l'ADN circulaire isolé par une transposase ;
c) amplifier *in vitro* l'ADN circulaire modifié comprenant de l'ADNec ou de l'ADNecc ; et
d) isoler l'ADNec ou l'ADNecc amplifié ;

dans lequel la réaction de transposition utilise un transposon qui est assemblé avec un ensemble d'amorces oligonucléotidiques d'ADN linéaires qui comprennent :

- SEQ ID : 1 et SEQ ID : 2 ; ou
- SEQ ID : 3 et SEQ ID : 4 ; ou
- SEQ ID : 5 et SEQ ID : 6.

9. Procédé selon la revendication 8, dans lequel :

a) l'origine de réplication est OriC *d'E. coli* ; ou
b) ladite pluralité d'ADN circulaire résulte d'une amplification par réaction de cycle de réplication (RCR) isotherme *in vitro.*

10. Procédé de production de la pluralité d'ADNec ou d'ADNecc selon la revendication 1, le procédé comprenant les étapes consistant à :

a) isoler l'ADN circulaire comprenant de l'ADNec ou de l'ADNecc à partir de la cellule ;
b) modifier l'ADN circulaire comprenant de l'ADNec ou de l'ADNecc en insérant l'origine de réplication procaryote dans l'ADN circulaire isolé par une transposase ;
c) appliquer une endonucléase Exo V pour éliminer l'ADN non circulaire linéarisé ;
d) appliquer PacI pour linéariser l'ADN mitochondrial circularisé ;
d) amplifier *in vitro* l'ADN circulaire modifié comprenant de l'ADNec ou de l'ADNecc ; et
e) isoler l'ADNec ou l'ADNecc amplifié ;

dans lequel la réaction de transposition utilise un transposon qui est assemblé avec un ensemble d'amorces oligonucléotidiques d'ADN linéaires qui comprennent :

- SEQ ID : 1 et SEQ ID : 2 ; ou
- SEQ ID : 3 et SEQ ID : 4 ; ou
- SEQ ID : 5 et SEQ ID : 6.

11. Procédé selon l'une quelconque des revendications 8 à 10, dans lequel l'ADN circulaire comprenant de l'ADNec ou de l'ADNecc est humain ;
éventuellement dans lequel :

a) l'ADNec ou l'ADNecc humain est isolé à partir d'un tissu ; éventuellement dans lequel le tissu est une tumeur ou une biopsie de celle-ci ; ou
b) l'ADNec ou l'ADNecc humain est isolé à partir d'un biofluide, éventuellement dans lequel le biofluide est du sang ou du liquide céphalo-rachidien (LCR).

12. Procédé d'identification d'une séquence d'acide nucléique d'ADN extrachromosomique circulaire (ADNec) ou (ADNecc) eucaryote, le procédé comprenant :

i) la réalisation d'un séquençage d'acide nucléique sur une composition selon la revendication 1 ; ou
ii) la réalisation d'un séquençage d'acide nucléique sur de l'ADN extrachromosomique circulaire (ADNec) ou (ADNecc) isolé à partir d'une cellule eucaryote, dans lequel l'ADN extrachromosomique circulaire (ADNec) ou (ADNecc) est obtenu selon le procédé de la revendication 8 ; ou
iii) la réalisation d'un séquençage d'acide nucléique sur de l'ADN extrachromosomique circulaire (ADNec) ou (ADNecc) isolé à partir d'une cellule eucaryote, dans lequel l'ADN extrachromosomique circulaire (ADNec) ou (ADNecc) est obtenu selon le procédé de la revendication 10.

13. Procédé selon la revendication 12, dans lequel :

a) le procédé inclut l'étape supplémentaire consistant à linéariser l'ADNec ou l'ADNecc amplifié avec Cas9 ou une enzyme de restriction avant le séquençage ; et/ou

b) le procédé d'identification d'une séquence d'acide nucléique est un séquençage à longues lectures par nanopore.

**14.** Procédé d'identification d'un patient cancéreux présentant une maladie métastatique pour un traitement, le procédé comprenant l'évaluation d'une composition selon la revendication 1 pour un ADNecc spécifique aux métastases.

**15.** Procédé d'identification d'un patient cancéreux présentant une maladie métastatique pour un traitement, comprenant les étapes consistant à :

a) isoler l'ADN extrachromosomique (ADNecc) à partir du patient ;

b) modifier l'ADNecc en insérant une origine de réplication procaryote dans l'ADN circulaire isolé par l'intermédiaire d'une réaction de transposition ;

c) appliquer une endonucléase Exo V pour éliminer l'ADN non circulaire linéarisé ;

d) appliquer PacI pour linéariser l'ADN mitochondrial circularisé ;

d) amplifier *in vitro* l'ADN circulaire modifié comprenant de l'ADNecc ;

e) purifier l'ADNecc amplifié ;

f) linéariser l'ADNecc amplifié avec une enzyme Cas9 ou une enzyme de restriction ;

g) caractériser le produit amplifié de l'étape g) en utilisant une technique de séquençage à longues lectures par nanopore ; et

h) utiliser la caractérisation de l'étape h) pour identifier un patient cancéreux présentant une maladie métastatique ;

dans lequel la réaction de transposition utilise un transposon qui est assemblé avec un ensemble d'amorces oligonucléotidiques d'ADN linéaires qui comprennent :

• SEQ ID : 1 et SEQ ID : 2 ; ou
• SEQ ID : 3 et SEQ ID : 4 ; ou
• SEQ ID : 5 et SEQ ID : 6.

# FIG 1

# FIG 2

# FIG 3

# FIG 4

# FIG 5

# FIG 6

# FIG 7

# FIG 8

# FIG 9

# Testing on normal cells: GM12878 ~ B-lymphocyte

# FIG 10

# FIG 11

# FIG 12

# FIG 13

Transposition/insertion

1310

1312

1304

Exo V

1307

PacI

circular DNA

Linear DNA

mitochondria DNA

1301

1302

1303

1306

1305

1300

Amplification

1313

1311

1315

ONT sample prep and sequencing

# FIG 14

# FIG 15

Read length distribution
(all reads)

Read length distribution
(all human genome hg38 aligned reads)

# FIG 16

Alignment to human genome (CHM13 T2T v1.1)

**FIG. 17**

| # | 1 | 2 | 3 | 4 | 5 | 6 | 7 |
|---|---|---|---|---|---|---|---|
| pUC19 | 1ng | 0.01ng | 0.0001ng | 1ng | 0.01ng | 0.0001ng | negative |
| Linear Lamda | 10ng | 10ng | 10ng | x | x | x | x |

**FIG. 18**

| | | PC3 | | PC3 + Plasmid | |
|---|---|---|---|---|---|
| | Sample # | 1 | 2 | 3 | 4 |
| | Input | 10ng | 1ng | 10ng | 1ng |
| *no PacI treatment | ExoV | + | + | + | + |

Mitochondria (about 16kb)

Plasmid 5.5kb

*Plasmid 5.5kb

*partial mitochondria

Linear DNA ladder

Super coiled DNA ladder

* Circular DNA at 2.5kb
    * Aligned to chr1
  * Repeats

**FIG. 19**

**FIG. 20**

**FIG. 21**

Plasmids — — Small circle

Mouse heart

circle — — Mitochondria

**FIG. 22**

| Plasmid | size-kb |
|---------|---------|
| pAT1359 | 9.13 |
| pAT1366 | 7.54 |
| pAT1023 | 5.44 |
| pBR322 | 4.36 |
| pUC19 | 2.69 |

**FIG. 23A**

- Preferential amplification of one plasmid

- Different plasmids amplified more evenly.

**FIG. 23B**

**FIG. 24**

**FIG. 25A**

FIG. 25B

FIG. 25C

PC3     pAT

**FIG. 25D**

transfection    -     +

mtDNA

pAT

??
Native eccDNA

**FIG. 25E**

HSAFM

**FIG. 25F**

**FIG. 25G**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63313549

- US 10301672 B

**Non-patent literature cited in the description**

- **WANG et al.** *Nature*, 2021, vol. 599, 308-314
- **FUNNEL et al.** *J Biol Chem.*, 1986, vol. 261, 5616-5624
- **ZHU et al.** *Sci Reports*, 2017, vol. 7, 10968
- **WANG et al.** *Nature*, 2021, vol. 599, 308-317
- **FAKRUDDIN et al.** *J Pharm Bioallied Scie.*, 2013, vol. 5, 245-252
- **PENG** ; **MARIANS**. *Proc. Natl. Acad. Sci. USA*, 1993, vol. 90, 8571-8575
- **HIASA et al.** *J. Biol Chem*, 1994, vol. 269, 2093-2099

- **HIASA et al.** *J. Biol Chem*, vol. 269, 26959-26968
- **MASAYUKI et al.** *Nucleic Acids Research*, 2017, vol. 45, 11525-11534
- **NARA** ; **MASAYUKI**. *BioTechniques*, 2021, vol. 71, 1-6
- **KAZUO et al.** *Front. Cell. Infect. Infect. Microbiol.*, 2020, vol. 10, 11
- **MASAYUKI et al.** *Nucleic Acid Res*, 2017, vol. 45 (20), 11525-11534